(19) Europäisches Patentamt

European Patent Office

Office européen des brevets

(11) Publication number: **0 296 982 B1**

## EUROPEAN PATENT SPECIFICATION

(45) Date of publication of patent specification: **15.09.93**　(51) Int. Cl.⁵: **A61F 9/00**

(21) Application number: **88401607.2**

(22) Date of filing: **24.06.88**

(54) **Device for correcting the shape of an object by laser treatment.**

(30) Priority: **25.06.87 FR 8708963**

(43) Date of publication of application:
**28.12.88 Bulletin 88/52**

(45) Publication of the grant of the patent:
**15.09.93 Bulletin 93/37**

(84) Designated Contracting States:
**DE FR GB**

(56) References cited:
**EP-A- 0 207 648
EP-A- 0 224 322
DE-A- 3 535 073
US-A- 4 648 400**

(73) Proprietor: **Hanna, Khalil**
**19, rue Las-Cazes**
**F-75007 Paris(FR)**

(84) Designated Contracting States:
**DE FR GB**

(73) Proprietor: **Compagnie IBM FRANCE**
**5 Place Vendôme**
**F-75000 Paris 1er(FR)**

(84) Designated Contracting States:
**FR**

(73) Proprietor: **International Business Machines Corporation**
**Old Orchard Road**
**Armonk, N.Y. 10504(US)**

(84) Designated Contracting States:
**DE GB**

(72) Inventor: **Khalil, Hanna**
**19, rue Las Cazes**
**FR-75007 Paris(FR)**
Inventor: **Asfar, Louis**
**8, Square de Bretteville**
**FR-78150 Le Chesnay(FR)**
Inventor: **Chastang, Jean-Claude**
**68, Hatfield Road - Box 354**
**R.F.D. 1 - Mahopac New York 10541(US)**

(74) Representative: **Martin, Jean-Jacques et al**
**Cabinet REGIMBEAU 26, Avenue Kléber**
**F-75116 Paris (FR)**

EP 0 296 982 B1

EP 0 296 982 B1

## Description

The present invention relates to a device for performing surgery on the cornea of the eye. The purpose of such modifications of the shape of the cornea is to correct ametropia by correcting dimensional optical characteristics of the cornea and principally its radius of curvature. At the present time such modifications, known as keratomileusis, are achieved by actually machining a disk removed from the cornea. The disk is made rigid by freezing it and then machined by the Barraquer process or applied to a template with the appropriate radius of curvature and recut using the Barraquer-Krumeich technique.

This type of operation has the major disadvantage of necessitating first removal of the disk of corneal material and then treatment of the aforementioned disk, which has to be reimplanted on the eyeball of the patient after treatment.

However, recent work has shown the very precise ablative properties of excimer laser radiation when this radiation is applied to the corneal tissue. The radiation emitted by an excimer laser, with a wavelength substantially equal to 193 nm, may be used to eliminate corneal material by photodecomposition. Generally speaking, a round light spot (an image of the laser beam) is formed on the cornea, the spot being substantially centered on the optical axis of the eyeball. The spot has a substantially circular or annular shape or a symmetrical shape relative to the optical axis of the eyeball and may be moved and/or the radius size changed, the exposure time for a particular area depending on the thickness of the cornea to be eliminated.

Although such devices enable direct operation on the eyeball of the patient, enabling better centering through avoiding the aforementioned problem of cutting out, and reimplanting after correction, a piece of the cornea, they do not make it possible to implement a precise treatment method in that, although the exposure time can be defined with good precision, the effects and in particular the thickness of the cornea subjected to photodecomposition vary with the size of the light spot and the energy density of the laser beam used. Moreover, the surface state of the cornea after treatment and undesirable side effects due to thermal or shockwave phenomena vary significantly with the energy level delivered by each pulse and the repetition frequency with which the same area is successively irratiated.

EP-A-0 207 648 discloses a system for ophtalmological surgery in which a cross section image of a laser beam is focused on to the corneal area to be treated. Successive openings of a masking plate are placed successively across the laser beam, in a fixed position in which each of them, successively, lies symmetrical with the optical axis ; the shapes of the successive openings are identical, i.e. circular, rectangular or elliptical, but their sizes incremently differ from one another, so that the cross section of the laser beam image on the cornea is varied for a single treatment operation, and the depth of irradiation of the focused beam is determined through the laser pulses number during illimination or by the duration of illumination in case continuous laser is used.

Since the dimensions of the cross section of the laser beam image are varied while the shapes and positions of the successive openings are identical, the mean power density focused on to the cornea at the level of the focused image of the laser beam has not a constant value.

DE-A-3 535 073 discloses a rotational diaphragm intended for controlling the depth at which a laser beam irradiates cornea, as a function of the thickness of the cornea, which is thicker at its periphery than at its center, taking into account the non-uniform incidence of the laser beam on the corneal surface, due to the curvature thereof.

In fact, DE-A-3-535 073 does not deal with the specific correction of different kinds of ametropy.

An object of the device in accordance with the present invention for performing surgery on the cornea of the eye using laser radiation is to remedy the aforementioned disadvantages through the use of a device enabling an ablation process to be carried out by successive discrete ablations, the total ablation resulting from the summation of numerous discrete ablations, while avoiding irradiating the same area with two or more consecutive pulses and limiting the surface area irradiated by each pulse.

Another object of the present invention is the use of a device in which each elementary discrete ablation is optimised both from the point of view of the extent of the area over which the discrete ablation is effected and the irradiation time for the area to carry out the aforementioned discrete ablation, the surface state of the area over which the discrete ablation has been effected featuring a minimum degree of roughness and the corrected area, the summation of the areas over which one or more discrete ablations have been effected, having a minimum degree of roughness, the reduction of undesirable side effects such as shockwave and thermal effects making it possible to preserve and respect the integrity of surrounding tissue.

Another object of the present invention is the use of a refractive surgery device for laser treatment of the cornea of the eye anabling direct operation on the eyeball of the patient, the operation being computer-

2

or microcompunter-assisted.

The present invention proposes a device for shaping the shape of an object by laser ablation of a surface of said object according to an ablation function, said device comprising :

. means for generating a pulsed laser beam having pulses and an energy density,

. slit means having at least one slit intercepting said laser beam, said at least one slit having a profile function proportional to said ablation function,

. means for forming an image of said at least one slit onto an area of said surface of said object,

. means for displacing said image of said at least one slit over said area by steps of a given increment, corresponding to elementary discrete ablations of said surface of said object,

. means for synchronizing said increment, said pulses and said energy density, so that the total ablation resulting from the summation of said elementary discrete ablations meets said ablation function.

The device in accordance with the invention finds an application in any surgical operation on the cornea of the eye intended to correct ametropia by keratomileusis in the case of myopia, hypermetropia and astigmatism, by epikeratothakia, by radiating incisions, bar-shaped incisions or circular incisions for corneal grafting, uniform deep ablation for lamellar grafting.

The invention will be better understood on reading the following description and referring to the drawings in which :

- figure 1 shows a graph plotting the depth of a discrete elementary ablation by one laser emission pulse as a function of the radiation energy density,
- figure 2a shows a plan view of the cornea of an eye with the corresponding definition of parameters defining the surface to be treated,
- figure 2b shows a view in cross-section on the line A-A in figure 2a with the corresponding definition of parameters defining the surface treated and the area removed by photodecomposition,
- figure 3a shows a block diagram of the device in accordance with the invention in the case where the image of the treatment laser beam is moved in rotation,
- figure 3b shows a particularly advantageous object slit enabling treatment by keratomileusis of myopia in the case of the embodiment of the device from figure 3a,
- figure 3c shows a particularly advantageous object slit enabling treatment by keratomileusis of hypermetropia in the case of the embodiment of the device from figure 3a,
- figure 3d shows in a non-limiting way one embodiment of an object slit with multiple lobes enabling treatment of myopia by keratomileusis in the same way as in the case of figure 3b,
- figures 3e and 3f respectively represent in an advantageous, non-limiting way an embodiment of an auxiliary slit of the circular sector type, enabling, when associated with an object slit such as that shown in figure 3b or figure 3c, treatment by keratomileusis of astigmatism of the eyeball and the cornea, in the case of the embodiment of the device from figure 3a and a circular incision for trepannation and for correction of astigmatism by partial and localised incisions,
- figure 4a shows a non-limiting alternative embodiment of the device in accordance with the invention shown in figure 3a in the case where the image of the treatment laser beam is moved either in rotation or in translation,
- figure 4b shows a particularly advantageous object slit enabling treatment of myopia by keratomileusis in the case of the embodiment of the device from figures 3a and 4a, the image of the laser beam being moved in translation,
- figure 4c shows a particularly advantageous object slit enabling treatment of hypermetropia by keratomileusis sin the case of the embodiment of the device from figure 4a, the image of the laser beam being moved in translation,
- figure 4d shows in a non-limiting way an alternative embodiment of an object slit with multiple lobes enabling treatment of myopia by keratomileusis in the same way as in the case of figure 3e,
- figure 4e shows a particularly advantageous embodiment in which at least one edge of the slit is adjustable to enable compensation of irregular distribution of the energy of the laser beam,
- figure 5a shows in the case of use of the device from figure 4a with the image of the laser beam moved in translation the area of the cornea subjected to irradiation in two elementary areas extending in two directions OX, OY, the areas defined by movement in translation of the laser beam in the corresponding direction OX or OY being concurrent,
- figure 5b shows a profile characteristic of total ablation of a cornea subjected to treatment for myopia by keratomileusis,
- figure 5c shows a profile characteristic of total ablation of a cornea subject to treatment for hypermetropia by keratomileusis,

- figures 6a and 6b show a non-limiting embodiment of a diaphragm enabling improved focussing of images of the slits onto the cornea and figures 6c through 6e show a particularly advantageous embodiment of a diaphragm the slit in which is moved in rotation, enabling discontinuity between the corrected and non-corrected areas of the cornea to be avoided,

- figure 7 shows an advantageous alternative embodiment of the device in accordance with the invention.

Prior to the description proper of the device for refractive surgical laser treatment of the cornea of the eye in accordance with the invention, there follow preliminary remarks summarising the effects of excimer laser light irradiation at a wavelength of 193 nanometres when such radiation is applied to the corneal tissue.

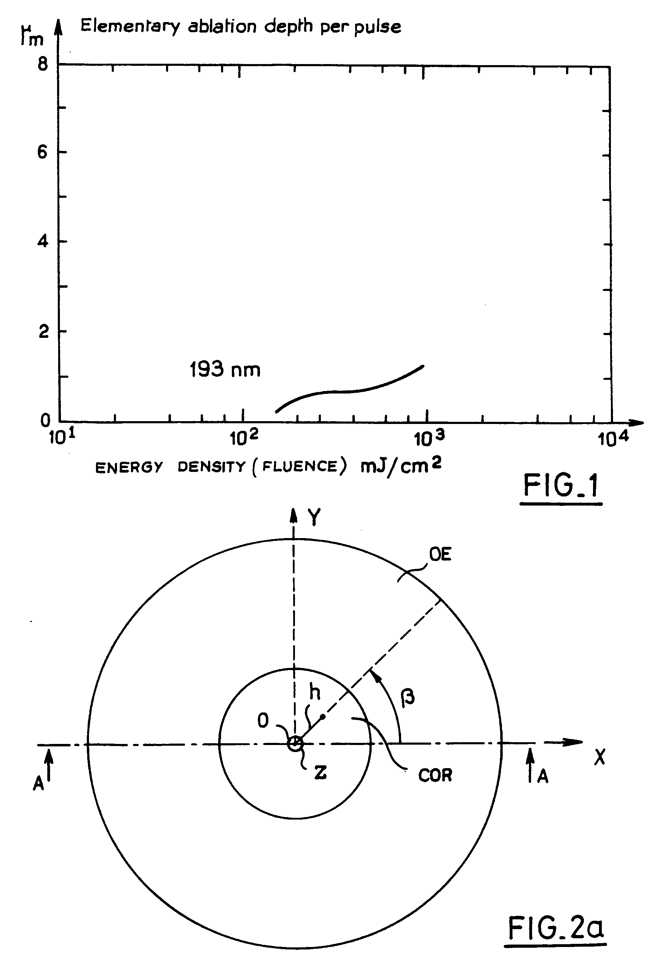

Figure 1 shows a curve of ablation on which the values of the depth of discrete elementary ablations are plotted on the ordinate axis, this axis being graduated in micrometres, as a function of the energy density per laser illumination pulse, the abscissa axis being graduated in millijoules/cm$^2$.

The discrete elementary ablation curve is characterised by the presence of a threshold, that is to say a value of the energy density below which no ablation occurs. Generally speaking, the curve is strongly non-linear and the depth of ablation increases only very slowly with the energy density. It will in fact be noted that the depth of each discrete elementary ablation is small, lying between 0.25 and 1 $\mu$m.

The refractive eye surgery device in accordance with the invention is, in its essentials, advantageously based on a discrete ablation process, a large number of discrete elementary ablations being employed to obtain a total resulting ablation. Although the discrete elementary ablation caused by a laser illumination pulse features the previously mentioned non-linearity with regards to its depth as a function of the energy density, it is assumed (providing that the energy density is constant from one pulse to another) that the resulting total ablation at a fixed point for a given number $n$ of consecutive pulses is equal to $n$ times the average ablation corresponding to a single pulse. Thus the discrete elementary ablation corresponding to the aforementioned average ablation is denoted:

$$\overline{a}(e) \qquad (1)$$

This average ablation corresponds substantially for a laser illumination pulse with an energy density in the order of 200 millijoules/cm$^2$ to a depth of ablation corresponding to the step in the curve shown in figures 1, and in practice to a depth of ablation between 0.5 and 0.8 $\mu$m.

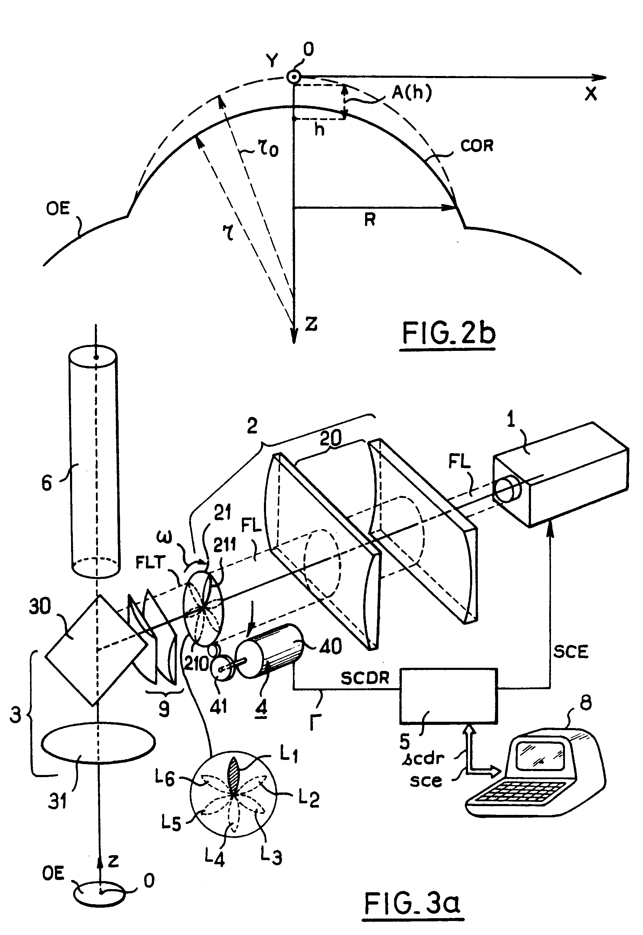

A more detailed description of the operations to be carried out to correct ametropia by correcting dimensional optical characteristics of the cornea and principally its radius of curvature will be given with reference to figures 2a and 2b. To simplify the description of the device in accordance with the invention, the principal operations aforementioned will be limited to keratomileusis for treating myopia, hypermetropia and myopic astigmatism.

Figure 2a shows a plan view of the eyeball designated OE. The aforementioned plan view is seen along the optical axis of the eye designated OZ in figure 2a, the aforementioned optical axis being centered on the cornea designated COR and the pupil of the iris, not shown in this figure. In the following explanation it will be considered that the optical axis and the visual axis of the eye are substantially coincident. Reference directions are denoted OX and OY, the frame of reference OX, OY being an orthogonal frame of reference. The distance from a given point on the corneal surface to the optical axis OZ is designated h.

Figure 2b shows a cross-section on the line A-A in figure 2a. In figure 2b the radius of curvature of the cornea COR before treatment, the cornea before treatment being shown in figure 2b in dashed outline, is designated $r_0$ while r designates the radius of curvature of the cornea COR after treatment using the device in accordance with the invention. Generally speaking, R designates the radius of the optical area on the cornea for operating on and correcting the latter. Of course, the value of this parameter R and the area of the cornea over which the operation will be carried out are defined by the practitioner, following a clinical analysis carried out by him or her. Finally, A(h) designates the ablation function, that is to say the thickness (in the direction Oz of the optical axis of the eye) to be removed by photodecomposition to a distance h from the optical axis OZ of the eye to alter the cornea from the initial radius of curvature $r_0$ to the final radius of curvature r, after the aforementioned operation.

In the case of keratomileusis for myopia, the object of the corresponding operation is to increase the radius of curvature of the cornea. The initial radius of curvature $r_0$ is increased to a value $r > r_0$ after the operation. This effect is obtained by ablation with a substantially parabolic profile of revolution and the ablation function is, using the notation from figures 2a and 2b:

$$A(h) = A_0 \frac{(1 - \frac{h^2}{R^2})}{} ; \quad 0 \leqslant h \leqslant R \tag{2}$$

In the case of keratomileusis for hypermetropia, the object of the operation is to reduce the radius of curvature of the cornea, the initial radius $r_0$ being reduced to a value of $r < r_0$. In this case ablation is still on a surface of revolution about the optical axis OZ of the eye, there being no ablation at the centre O, for $h = 0$, and maximum ablation for a particular value $h = \nu$. The ablation of the corneal profile between $h = \nu$ and R then constitutes a merging area defined by purely mechanical considerations: no sudden transition with the optical area proper ($h < \nu$) or with the rest of the cornea ($h > R$). The ablation function A(h) satisfies the equation:

$$A(h) = A_0 \frac{h^2}{R^2} \quad \text{where} \quad 0 \leqslant h \leqslant \nu \tag{3}$$

For values of $\underline{h}$ greater than $\nu$ and less than R, the ablation function A(h) is a polynomial in $\underline{h}$ defining the aforementioned merging area according to previously mentioned mechanical considerations.

In equations (2) and (3) above, $A_0$ represents, of course, the extent of ablation for $h = 0$, that is the thickness of ablation at the optical axis OZ of the eye itself:

$$A_0 = \frac{R^2}{2} \left( \frac{1}{r_0} - \frac{1}{r} \right)$$

In the case of keratomileusis for myopic astigmatism, the ablation is no longer on a surface of revolution. It will be remembered that in cases of corneal astigmatism the principal astigmatism directions are defined by orthogonal planes in which it is possible to define a maximum radius of curvature and a minimum radius of curvature for the optical surface in question, in this instance the cornea. In this case, and by way of simplification, and in line with what the practitioner will have to do in any event to carry out the operation using the device in accordance with the invention, it is advantageous to take as the reference directions OX and OY the principal astigmatism directions as previously defined. The aforementioned directions OX and OY are then contained in the af“ormentioned astigmatism planes. The radius of curvature of the cornea COR is in this case a function of the azimuth angle denoted $\beta$, the radius of curvature $\underline{r}$ of the cornea after the operation for example satisfying the equation:

$$r(\beta) = r_x \cos\beta + r_y \sin\beta \tag{4}$$

In equation (4), $\beta$ represents the azimuth angle of any plane containing the optical axis OZ, the azimuth angle being for example the dihedral angle formed by the aforementioned any plane and the plane OZ, OX. The values $r_x$ and $r_y$ are the corresponding values of the radius of curvature $\underline{r}$ for $\beta = 0$ and $\beta = \pi/2$, respectively.

In the case of keratomileusis for myopic astigmatism, research has shown that the ablation profile may be written (the OX and OY axes having been determined as previously described):

$$A(X,Y) = A_0 \left( 1 - \frac{X^2}{R_X^2} - \frac{Y^2}{R_Y^2} \right) \tag{5}$$

In equation (5), the quantities $A_0$, $R_x$ and $R_y$ are defined by:

$$A_0 = \frac{A^x_0 + A^y_0}{2}, \quad R_x = R\sqrt{\frac{A^0}{A^x_0}}, \quad \text{and} \quad R_y = R\sqrt{\frac{A^0}{A^y_0}} \qquad (6)$$

The terms $A^x_0$ and $A^y_0$ are themselves defined as functions of the parameters R, $r_x$ and $r_y$ by equations (7) and (8) below:

$$A^x_0 = \frac{R^2}{2}\left(\frac{1}{r_x} - \frac{1}{r}\right) \qquad (7)$$

$$A^y_0 = \frac{R^2}{2}\left(\frac{1}{r_y} - \frac{1}{r}\right) \qquad (8)$$

Generally speaking, iso-ablation curves are ellipses.

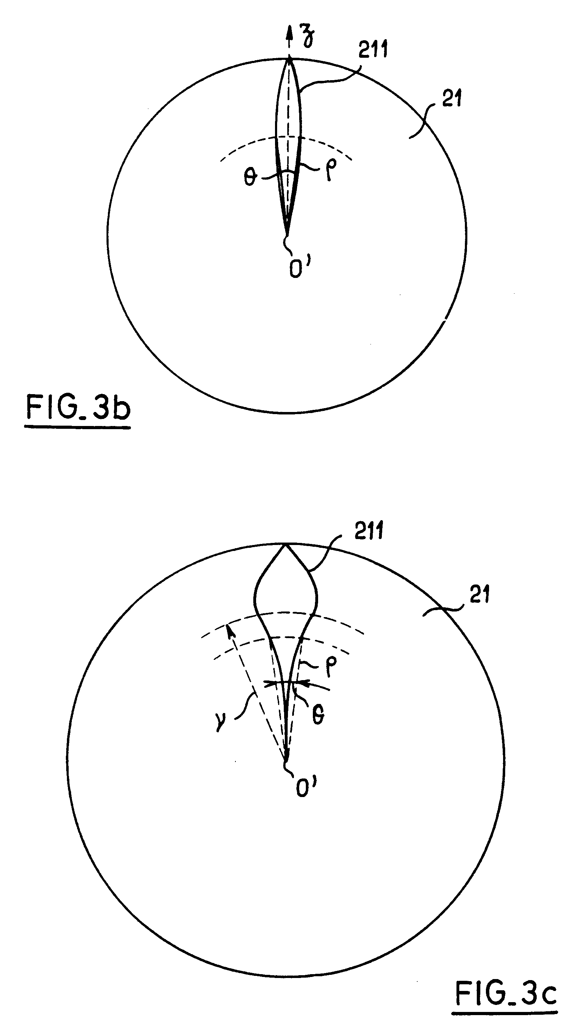

A more detailed description of the device in accordance with the invention for performing refractive surgery on the eye by laser treatment of the cornea will now be given with reference to figure 3a.

Referring to the aforementioned figure, the device in accordance with the invention comprises means 1 for emitting a laser beam denoted FL. The laser beam FL is a pulsed laser beam.

The means for emitting the laser beam FL are preferably an excimer laser emitting radiation at a wavelength of 193 nanometres. The emission means 1 preferably emit laser pulses with an energy level of the laser beam FL in the order of 180 millijoules per pulse, the repetition frequency of the laser pulses being in the order of 20 Hz. The duration of each pulse is in the order of 10 nanoseconds and the instantaneous power of each pulse reaches high values, in the order of 10 MW.

As further seen in figure 3a, the device in accordance with the invention comprises means 2 for generating a treatment laser beam denoted FLT comprising at least one lobe denoted L1 through L6 of elongate cross-section. In figure 3a the image of the treatment laser beam FLT has been shown to a larger scale, it being possible to show this image on a screen, for example, not shown in figure 3a.

The device in accordance with the invention also comprises means 3 for focussing the image of the lobe or lobes L1 through L6 of the treatment laser beam FLT on the area of the eye OE to be corrected, on the cornea of the latter. Of course, the means 2 for generating the treatment laser beam FLT and the means 3 for focussing the image cause a loss of energy of the laser pulses of the laser beam FL, but the energy delivered to the cornea COR is in the order of 5 millijoules per pulse. The energy density on the image of the lobes of the laser beam generated by the means 3 for focussing the image of the aforementioned lobes is in the order of 200 millijoules/cm$^2$ as previously explained.

According to an advantageous aspect of the device in accordance with the invention, means 4 for moving the image of the lobe or lobes of the treatment laser beam FLT are provided for moving the aforementioned image over the area of the eye OE to be corrected.

Means 5 for synchronising the displacement of the image of the lobe or lobes of the treatment laser beam FLT over the area of the eye to be corrected are provided to ensure synchronisation with the pulses of the treatment laser beam.

Although the precise mechanism of the ablation process is still the subject of research, in some aspects it may be regarded as similar to a micro-explosion causing by photodecomposition a discrete elementary ablation by each laser pulse. The total correction or ablation resulting from implementation of a method of using the device of the invention is effected by summation of a plurality of elementary discrete ablations.

According to another advantageous characteristic of the device in accordance with the invention shown in figure 3a, the means 3 for focussing the image of the lobe or lobes L1 through L6 of the treatment laser beam FLT make it possible to focus the aforementioned image in such a way that the generatrix of an end of the lobe or lobes or the axis of longitudinal symmetry of the aforementioned lobe or lobes of the treatment laser beam are coincident with the optical axis OZ of the eye to be treated. Of course, as shown in figure 3a, the device in accordance with the invention may advantageously comprise an alignment device denoted 6 consisting, for example, of an auxiliary laser emission device such as a low-power helium-neon laser enabling the practitioner to carry out the appropriate adjustments of the focussing means 3 relative to the optical axis OZ of the eye OE of the patient.

According to another advantangeous characteristic of the device in accordance with the invention, the means 4 for displacing the image of the lobe or lobes of the treatment laser beam over the area of the eye to be corrected make it possible to displace the image of the aforementioned lobes L1 through L6 in rotation about the previously mentioned end generatrix or the longitudinal axis of symmetry of the lobe or lobes of the treatment laser beam FLT.

According to an advantageous aspect of the device in accordance with the invention, the latter enables the aforementioned rotation by increments of the angle of rotation denoted $\Gamma$.

In one specific embodiment of the device in accordance with the invention shown in figure 3a, the means 2 for generating the treatment laser beam FLT may advantageously comprise a focussing optical system 20. The focussing optical system 20 may consist of a Galilean telescope producing from the laser emission means 1 a laser beam FL of regular (for example cylindrical) cross-section.

According to another particularly advantageous aspect of the device in accordance with the invention, the means 4 for displacing the image of the lobe or lobes of the treatment laser beam in rotation may comprise, as shown in figure 3a, a mask or diaphragm 21 incorporating an object slit denoted 211. Of course, the object slit 211 is of elongate shape and illuminated, for example in parallel light, by the laser beam FL. One end of the object slit 211 is disposed, for example, at the centre of the diaphragm 21 and generates the aforementioned end generatrix of the treatment laser beam FLT or the longitudinal axis of symmetry of the lobes L1 through L6 of the treatment laser beam FLT.

The object slit 211 and the image of this object slit are rotated by drive means 40, 41 for rotating the mask or diaphragm 21.

Of course, but not in any limiting way, the diaphragm 21 may be a circular shape diaphragm and the drive means for the diaphragm 21 advantageously comprise a toothed ring denoted 210 disposed at the periphery of the diaphragm and a stepper motor 40 the drive shaft of which is fitted with at least one toothed wheel 41 meshing with the toothed ring 210.

To focus the image of the lobe or lobes of the treatment laser beam FLT, the focussing means 3 advantageously comprise a semi-reflecting mirror 30 consisting of a prism or the like, for example, serving by total reflection to transmit the treatment laser beam FLT and the alignment beam delivered by the alignment means 6, together with a focussing lens 31 constituting the objective lens of the device. The combination of the semi-reflecting mirror 30 and the focussing lens 31 serves to form the image of the treatment laser beam FLT on the area of the cornea to be treated, of course.

In a conventional way, all of the device in accordance with the invention and in particular the means 2 for generating the treatment laser beam FLT and the laser emission means are mounted on an optical bench and the focussing means 3 are mounted on a barrel that can be oriented by the practitioner for correct aiming onto the area of the eye to be treated. The corresponding mountings for the aforementioned component parts as a whole will not be described as they constitute part of the prior art in the field of high-precision optical instruments.

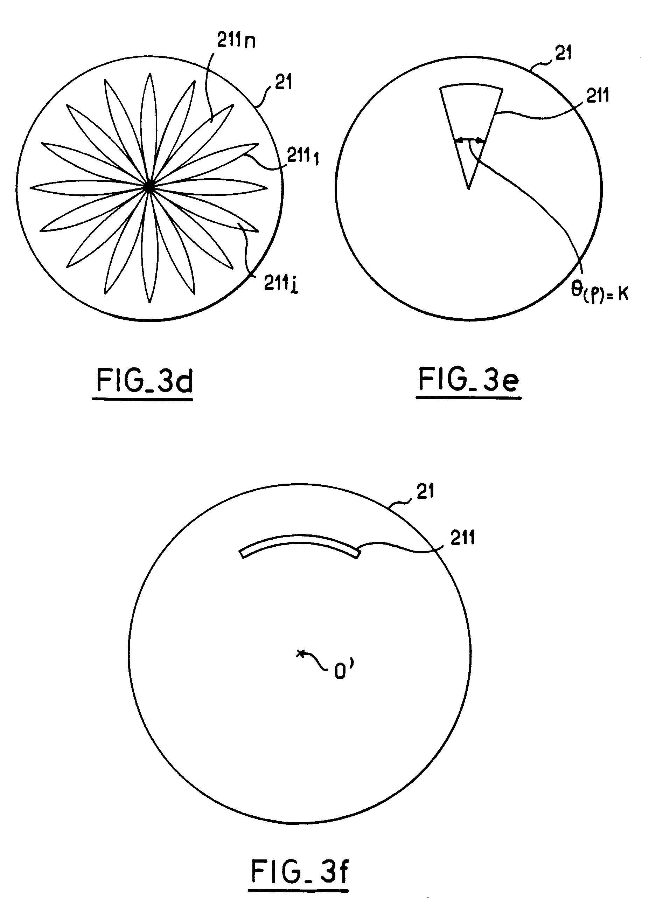

A more detailed description of the diaphragm enabling operations as previously described herein by means of the image of the laser beam lobe moved in rotation over the area of the eye to be treated will now be given with reference to figures 3b, 3c, 3d and 3e.

One embodiment of the object slit 211 of the diaphragm 21 will be described first in connection with treatment or operation by keratomileusis for myopia, the image of the lobe or lobes of the treatment laser beam FLT being rotated about the optical axis OZ of the eye to be treated.

Referring to the aforementioned figure 3b, the object slit 211 of the diaphragm 21 has a profile satisfying the equation:

$$\Theta(\rho) = \Gamma \frac{A_0}{\overline{a}(e)} \left(1 - \frac{\rho^2}{R^2}\right) = \Theta(0)\left(1 - \frac{\rho^2}{R^2}\right) \qquad (9)$$

In the aforementioned equation, $\theta(\rho)$ represents the aperture angle of the slit defined as the angle at the centre of a circle with its centre at the end of the object slit, for generating the end generatrix or the axis of symmetry of the treatment laser beam FLT with for radius the corresponding value $\rho$ of the distance from a point on the edge of the slit or lip of the object slit or of the lobe of the laser beam to the aforementioned centre.

In figure 3b it will be noted that the object slit 211 has convex lips or edges, the aperture angle $\theta(0)$ of the slit at the origin, that is to say at the centre O′ at the end of the slit being maximum.

$\Gamma$ represents the increment of angular rotation as previously mentioned. It will be noted that equation (9) represents the equation in polar coordinates of one of the lips of the slit, the other being deducible by considerations of symmetry.

Another example of an embodiment of an object slit 211 of the diaphragm 21 for treatment of hypermetropia by keratomileusis in the case where the operation is conducted by rotating the image of the lobe or lobes of the treatment laser beam FLT will also be described with reference to figure 3c.

In this case, as shown in the aforementioned figure, the profile of the slit 211 satisfies the equation:

$$\Theta(\rho) = \Gamma \, \frac{A_0}{\overline{a}(e)} \, \frac{\rho^2}{R^2}, \quad \rho \, \epsilon \, [0, \, \nu], \quad \nu < R \qquad (10)$$

$$= \theta_{max} \, \frac{\rho^2}{R^2} \quad \text{where} \quad \theta_{max} = \Gamma \, \frac{A_0}{\overline{a}(e)}$$

In equation (10) the parameters are defined according to the definitions previously given. It will be noted that the lips of the slit 211 in the case of figure 3c are substantially concave up to a particular value of the radius $\rho$, this particular value being denoted $\nu$. it will be noted that the corresponding lip then has a point of inflection, the curvature of the latter becoming convex and decreasing regularly up to the end of the slit corresponding to the maximum logitudinal dimension of the latter. This continuous decrease in the aperture angle $\theta$ beyond the value of the radius $\rho = \nu$ advantageously serves to prevent excessive discontinuity at the periphery of the resulting total ablation. In a non-limiting way and by way of example only, the particular value of $\nu$ is substantially equal to 2/3 of the maximum longitudinal dimension of the slit.

Of course, as shown in figure 3d in particular, the diaphragm 21 may advantageously comprise a plurality of elementary object slits denoted $211_1$, $211_i$ through $211_n$ in the aforementioned figure. Each elementary object slit generates a corresponding lobe of the treatment laser beam FLT, of course. The number of slits in the same diaphragm 21 is limited only by the maximum aperture $\theta_{max}$ of the object slit in question, the aperture angle at the origin $\theta(0)$ of each slit in the case of figure 3d and $\theta(R)$ in the case of figure 3c, for treatment of hypermetropia by keratomileusis.

It will be noted, of course, that increasing the number of object slits on the diaphragm provides for a commensurate decrease in the total operation time, since the summation of the successive elementary ablations achieved on the area to be treated by rotating the diaphragm and the object slit is added to the spatial summation due to the corresponding distribution of the various object slits on the diaphragm. It will be noted that in the case of multiple slits they may be regularly distributed over the diaphragm and all meet at their common end situated of the axis of rotation. Each of the slits generates in this way one lobe of the treatment laser beam FLT. In the case of slits used for treatment of myopia by keratomileusis, adjacent slits tangential to the centre have a surface area exactly equal to one-half the surface area of the disk within which the slits are inscribed.

It will be noted that the choice of the angular rotation increment $\Gamma$ actually determines the surface area of the object slit or slits used and vice versa. The choice of the angular increment $\Gamma$ and the maximum aperture angle $\theta_{max}$ are governed by the following considerations:

A narrow slit corresponding to a small angular increment $\Gamma$ enables use of a small part of the laser beam FL with the possibility of choosing the most homogeneous part of the latter, use of a low-power laser and also irradiation of a small part of the cornea by each pulse. Furthermore, increasing the number ND of slit images that are totally separated or at worst tangential, the number of images ND being denoted $ND_1$ in the case of treatment of myopia by keratomileusis and $ND_2$ in the case of treatment of hypermetropia by keratomileusis, means that the sequence of positions of the irradiated slits can be programmed to minimise heating of the cornea.

On the other hand, too small a rotation increment $\Gamma$ can lengthen the correction or treatment period.

In practice it is more advantageous to have a limited set of slits and to vary the rotation increment $\Gamma$ as appropriate to the required correction.

Thus a slit is totally defined by:

- its length which defines the radius of the corrected area, that is to say the parameter R defined by the practitioner,
- the type of correction or operation carried out, that is to say keratomileusis for myopia or hypermetropia,
- the maximum aperture angle $\theta$max appropriate to the type of correction or operation carried out.

For optimum performance of the operation, the device in accordance with the invention comprises means 8 for calculating the angular rotation increment $\Gamma$ which, for a given object slit (the slit having been chosen beforehand by the practitioner, of course) satisfies the equation:

$$\Gamma = \theta_{max} \frac{\overline{a}(e)}{A_0} \qquad (11)$$

The calculation means 8 are then used to determine the number of laser emission pulses NI, this number of laser pulses being denoted $N1_1$ in the case of treatment of myopia by keratomileusis. The number $NI_1$ of laser emission pulses satisfies the equation:

$$N_{I1} = \frac{2\pi}{\Gamma} = N_{D1} \frac{A_0}{\overline{a}(e)} \qquad (12)$$

In the aforementioned equation $ND_1$ represents the number of separate or adjacent slit images that can be formed on the area of the cornea COR to be treated.

The calculation means 8 are also used to calculate the minimum total irradiation time denoted $T_{min}$ or $T_{1min}$ in the case of treatment of myopia by keratomileusis. In this case, the minimum total irradiation time satisfies the equation:

$$T_{1min} = N_{I1} \frac{\tau(e)}{N_{D1}} = \tau(e) \frac{A_0}{\overline{a}(e)} \qquad (13)$$

In this equation, $\tau(e)$ represents the minimum time interval between two successive irradiations of the same point on the cornea. The value of $\tau(e)$ is established experimentally and is the threshold beyond which heating of the cornea may occur. The value $T_{1min}$ depends of course on the energy flux but does not depend on the rotation increment $\Gamma$. This is because all of the $ND_1$ separate slits can be irradiated in the aforementioned interval $\tau(e)$. In practice, the type of laser used to produce the laser pulses and the maximum speed of displacement of the slit may limit the frequency at which the pulses can be delivered.

The refractive eye surgery device using laser illumination in accordance with the invention may also be used to correct astigmatism of the cornea COR or of the eyeball.

In a case like this the ablation profile varies with the meridian in question of the eyeball, this meridian consisting of the intersection with the surface of the cornea of a plane containing the optical axis OZ of the eyeball oriented at an angle $\beta$ in azimuth relative to a plane containing the previously defined reference direction OX. In the case where, as previously defined, the reference directions OX and OY correspond to the principal directions of astigmatism, and in the case of myopic astigmatism, the ablation function satisfies the equation:

$$A(h, \beta) = A_0(\beta) \quad (1 - \frac{h^2}{R^2}) \qquad (14)$$

In this equation, $A_0(\beta)$ is equal to:

$$A_0(\beta) = \frac{1}{2} R^2 \left(\frac{1}{r(\beta)} - \frac{1}{r}\right) \qquad (15)$$

From the equations (6), (7) and (8) previously given in this description, it is possible to compensate for the variations in $A_0(\beta)$ by varying the rotation increment $\Gamma$ as a function of $\beta$.

In this way it is possible to correct astigmatism of the eyeball with slits identical to those previously described with reference to figures 3a, 3b, 3c, 3d by modulating the angular rotation increment $\Gamma$ as a function of the angle $\beta$ defining the meridian of the cornea of the eyeball.

To this end, the device in accordance with the invention comprises means for modulating the angular rotation increment $\Gamma$ as a function of the angle $\beta$, this angle rotation increment $\Gamma$ as a function of the angle $\beta$ satisfying the equation:

$$(\beta) = \Theta_{max} \frac{\bar{a}(e)}{A_0(\beta)} \qquad (16)$$

In this equation, $A_0(\beta)$ represents the ablation at the origin near the optical axis OZ of the eyeball in the direction with azimuth angle $\beta$.

However, in the case of myopic astigmatism, the ablation at the centre is not constant and varies with the meridian. To establish circular symmetry of the cornea the device in accordance with the invention may comprise as shown in figure 3e at least one auxiliary diaphragm 21 provided with an object slit 211 of circular sector shape the equation for which in polar coordinates is $\theta(\rho) = k$ where $k$ is a constant. The aforementioned auxiliary slit 211 enables such correction by means of supplementary irradiation and rotational displacement by the rotation increment $\Gamma(\beta)$ modulated as a function of the azimuth angle $\beta$ to establish a constant ablation at the origin 0 without modifying the radius of curvature of the cornea, however. The residual ablation to be effected during such supplementary irradiation using the slit 211 shown in figure 3e satisfies the equation:

$\delta A_{(\beta)} = A_0 (0) - A_0 (\beta)$

with

$$A_0 (0) = \frac{R}{2} \left(\frac{1}{min (rx, ry)} - \frac{1}{r}\right) )$$

in which equation min (rx,ry) represents the smaller of the values rx and ry.

The residual ablation effected during the supplementary irradiation is then obtained by modulating the angular rotation increment $\Gamma$ as a function of the azimuth angle $\beta$, the rotation increment $\Gamma$ satisfying the equation:

$$\Gamma(\beta) = \Theta(0) \frac{\bar{a}(e)}{\delta A(\beta)} \qquad (18)$$

It should be noted that this method introduces a discontinuity at the periphery of the resulting total ablation, this discontinuity being null for $\beta = 0$, that is in the OX direction, and maximal for $\beta = \pi/2$, that is

in the OY direction. The maximal value of this discontinuity is equal to:

$$\frac{R^2}{2} \left( \frac{1}{ry} - \frac{1}{rx} \right) \qquad (19)$$

with rx < ry.

This discontinuity can be resolved, as will be explained later in this description.

In an alternative embodiment of the device shown in figure 3a, for the purpose of compensating by correction astigmatism of the eyeball and of the cornea, the device may comprise upstream of the focussing means 3, on the path of the treatment laser beam FLT, an anamorphic optical system 9 in which the magnification depends on the azimuth angle $\beta$. In this case, the iso-ablation curves on the cornea are ellipses. Correction of astigmatism implies that the total resulting ablation as a function of the aximuth angle $\beta$ is not constant. Anamorphic systems are systems in which the magnification depends on the aforementioned azimuth angle $\beta$. Generally speaking, and with the orientation of the axes OX and OY previously defined relative to the eyeball in figure 2a, an anamorphic system having a corresponding magnification denoted $M_x$ and $M_y$ at an elementary surface dS of the object, that is to say of the object slit 211, corresponds to an elementary surface $dS' = M_x.M_y.dS'$ of the image given by the anamorphic system. Under these conditions, the image of a circle obtained by means of the rotating slit or by some other equivalent means is an ellipse. Thus the iso-energy curves in the object plane of the anamorphic system, that is to say of the object slit 211, are circles and the images of these circles given by the anamorphic system are ellipses. Given that the total resulting ablation at a given point on the cornea is proportional to the energy received at that point, the iso-ablation curves are consequently ellipses. $R_x$ and $R_y$ being the half major axes of these ellipses, the magnifications $M_x$ and $M_y$ of the anamorphic system 9 must be in the same ratio as the aforementioned half major axes. The anamorphic system 9 may consequently comprise two cylindrical lenses the longitudinal axes of which are orthogonal and respectively oriented to define the corresponding directions OX and OY, the lenses having respective magnifications $M_x$ and $M_y$. These anamorphic optical systems as such are prior art and because of this they will not be described in more detail in this description.

Of course, to facilitate the work of the practitioner the device in accordance with the invention may be provided with an auxiliary diaphragm 21 having an object slit 211 of circular arc shape with a particular radius of curvature. This type of object slit is shown in figure 3f by way of non-limiting example. It is used to make circular incisions for arc-shaped corneal grafts, for example.

Also, the object slit as shown in figure 3e may also be used to correct astigmatism as previously described by modulating the rotation increment as a function of the azimuth angle $\beta$, to carry out such operations as removal of a locally parallel faced meniscus for epikeratothakia, or removal of a parallel surface corneal disc from a donor or removal of a surface to be modified by the laser for correcting myopia or hypermetropia, with a view to carrying out lamellar grafting. The lamellar grafting operations may then be carried out with constant rotation increments $\Gamma$, the ablation obtained during this operation corresponding to that of a locally parallel faced meniscus the edges of which are substantially rectilinear.

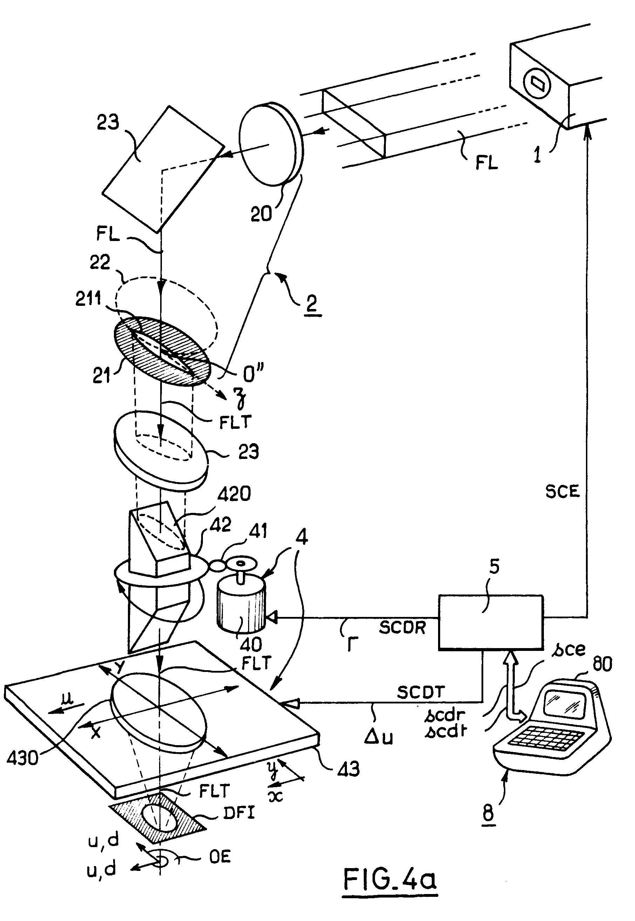

An alternative embodiment of the device in accordance with the invention more particularly adapted to operations such as those previously described will be described with reference to figure 4a.

In the embodiment shown in the aforementioned figure, but in a non-limiting way, the means 4 for displacing the image of the lobe or lobes of the treatment laser beam FLT over the area of the line to be treated provide for displacement in translation in a direction d substantially perpendicular to the largest dimension denoted Oz of the lobe of the treatment laser beam FLT. In this case, as will be described in more detail later in this description, the treatment laser beam FLT may be advantageously comprise two lobes or component parts of a single lobe symmetrical relative to a centre of symmetry denoted O″.

According to an advantageous characterstic of the device in accordance with the invention shown in figure 4a, the displacement in translation is advantageously effected by means of displacement increments denoted $\Delta u$. The displacement in translation is defined relative to the two reference directions OX, OY with u = X or u = Y, these directions defining a plane tangential to the cornea at the point O on the optical axis of the eyeball as defined previously in figure 2a.

The means 4 for displacement in translation of the image of the lobe or lobes of the treatment laser beam FLT advantageously provide for displacement in translation of the latter in the orthogonal directions OX and OY.

EP 0 296 982 B1

As shown by way of non-limiting example in figure 4a, the means 4 for displacing the image of the lobe or lobes of the treatment laser beam FLT in translation may comprise in succession along the path of the laser beam FL: a fixed diaphragm denoted 21 comprising at least one object slit 211 of elongate shape. This object slit is illuminated with parallel light. As shown in a non-limiting way in figure 4a, the laser beam FL may be generated by the means 1 previously described in relation to figure 3a, the laser beam FL possibly having a rectangular cross-section obtained in the classical way by passing the emitted laser beam through suitable diaphragms. Of course, as shown in figure 4a, a lens 20, a direction-changing mirror 21 such as a semi-reflecting mirror enabling under conditions analogous to those of figure 3a transmission of an auxiliary alignment laser beam not shown in this figure and a field lens 22 are used to conduct the parallel light laser beam FL to the slit 211 in the diaphragm 21.

Moreover, as also shown in figure 4a, a first lens 23 is placed relative to the object slit 211 and to the diaphragm 21 so that the object slit 211 is in the object focal plane of the lens 23 to generate the lobe or lobes of the beam imaging the object slit in parallel light.

A rotating prism 420 is provided whereby rotation of the prism in question through an angle $\alpha$ rotates the emergent light beam, i.e. the treatment laser beam FLT, through an angle $2\alpha$.

Also, a second focussing lens 430 serving as an objective lens is movable in translation in the previously mentioned directions OX and OY.

It will be understood that the embodiment of the device in accordance with the invention shown in figure 4a is particularly advantageous in that it enables two methods to be used: in the first the treatment laser beam FLT is scanned in rotation, the focussing lens 430 being held in a fixed position and centred on the optical axis OZ of the eye, of course, the prism 420 then being rotated to obtain the corresponding scanning of the treatment laser beam; in the second method, with the prism 420 fixed in position, the treatment laser beam emerging from the prism 420 is directed along the optical axis OZ of the eye and the focussing lens 430 produces corresponding movement in translation of the treatment laser beam FLT by corresponding defocussing due to movement of the lens 430 in translation in direction X or in direction Y.

The rotator prism 420 may advantageously be a Dove or Wollastom prism. Also, a diaphragm denoted DFI may be provided between the lens 430 and the eye of the patient to limit the luminous intensity received by the eye OE of the patient. It may be disposed in the vicinity of or on the eye. Of course, other direction-changing mirrors can be provided on the path of the laser beam FL to obtain an appropriate optical path to enable unrestricted circulation of persons in the environment of the apparatus and the practitioner.

The device in accordance with the invention in figure 4a is particularly advantageous in that, over and above any possible operation by scanning the area of the eye to be treated in rotation, it also makes it possible to carry out this operation by scanning the laser beam over the area of the eye to be treated in translation, in particular in the previously mentioned two directions OX and OY. The lobe or lobes of the laser beam and the beam direction Oz being oriented in the OY direction, the scanning in one direction (the OX direction, for example) is obtained by means of the rotator prism 420. This orients the aforementioned direction Oz with the OX direction for subsequent movement of the treatment laser beam FLT in the direction perpendicular to the new orientation of the Oz axis, i.e. the direction OY. The displacement in translation is effected by displacing the lens 430 in the corresponding directions.

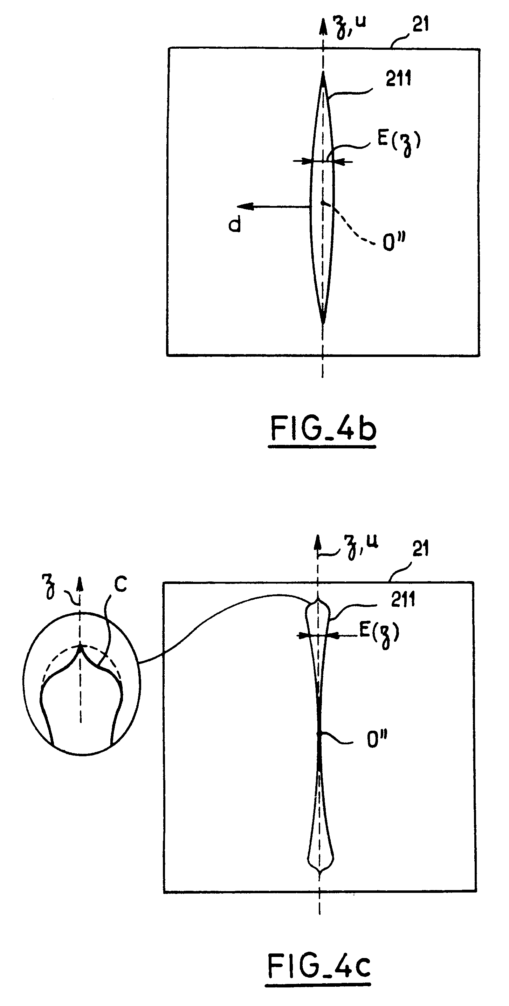

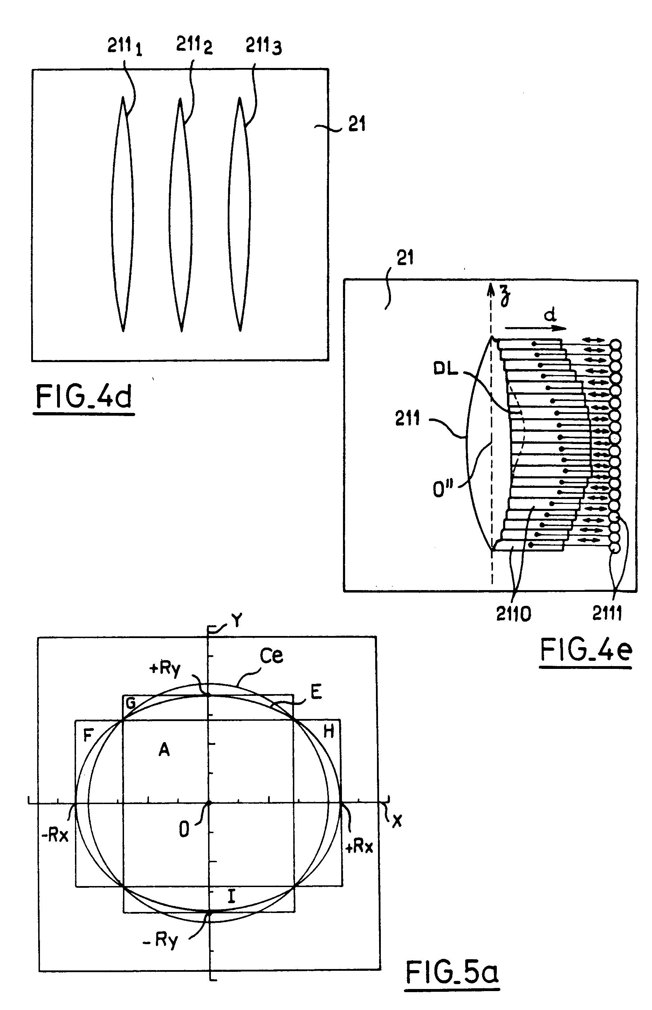

A more detailed description of an object slit 211 profile specifically used in the case where displacement in translation of the image of the object slit 211 is brought about to carry out the treatment or operation as aforementioned will be given with reference to figures 4b, 4c, 4d.

Referring to figure 4b, the object slit 211 of the diaphragm 21 and consequently the image of the lobe or lobes of the treatment laser beam FLT for treatment and correction by keratomileusis of myopia and astigmatism has a substantially parabolic profile. The profile defined by one lip of the slit satisfies the equation:

$$E(z) = 2 E_{max} \left( \frac{1}{2} - \frac{z^2}{R^2} \right) \qquad (20)$$

It will be noted for convenience that the slit 211 has a longitudinal axis denoted O″x.

In the above equation, the various parameters are defined as follows:

E(z) represents the transverse dimension of the object slit or of the lobe of the treatment laser beam at the abscissa z on the longitudinal reference axis oriented relative to the slit. The abscissa is referenced

12

relative to an origin point O″.

$E_{max}$ represents the maximal transverse dimension of the object slit 211.

While carrying out the aforementioned operation, the practitioner is required to displace the image of the object slit 211 in translation along a direction at least perpendicular to the longitudinal axis O″z of the object slit 211. Of course, the image of the object slit 211 is then oriented in such a way that the longitudinal axis O″z of the latter is oriented in one of the directions OX or OY of figure 2a. Thus for a direction $\underline{u}$ of orientation of the slit 211 or of its longitudinal axis O″z in the direction OX or OY, the equation relating the aperture of the slit E(u) and the translation displacement increment denoted Δu, this displacement being in the direction perpendicular to the orientation direction $\underline{u}$ of the slit, is of the form:

$$E(u) = \Delta_u \, \frac{A_0}{\overline{a}(e)} \, (\frac{1}{2} - \frac{u^2}{R^2}) \qquad (21)$$

In this equation:

u represents the abscissa or position of the edge of the slit on the longitudinal axis of reference O″z, the slit itself being oriented in the direction $\underline{u}$ corresponds to the direction OX or to the direction OY,

Δu represents the translation displacement increment in the direction orthogonal to the aforementioned alignment direction $\underline{u}$, i.e. in the direction OY or in the direction OX,

$A^u_0$ represents the thickness of ablation or correction at the centre of the area of the cornea to be corrected at the time of displacement in translation of the object slit 211 or of the lobe of the treatment laser beam in the direction OY or in the direction OZ.

A description of an object slit 211 for treatment and correction of the cornea by keratomileusis for hypermetropia and hypermetropic astigmatism will also be given with reference to figure 4c.

In the case of the aforementioned operation, the object slit 211 and the corresponding lobe or lobes of the treatment laser bean FLT have a substantially parabolic profile satisfying the equation:

$$E(z) = Emax \, (\frac{z^2}{R^2}) \qquad (22)$$

As in figure 4b the orientation of the longitudinal axis O″z of the object slit 211 in the direction OX or in the direction OY serves to establish the relationship defining the connection between the displacement increment Δu in the direction perpendicular to the orientation direction and the aperture E(u) of the slit 211, this relationship being of the form:

$$E(u) = \Delta u \, \frac{A^u_0}{\overline{a}(e)} \, (\frac{u^2}{R^2}) \qquad (23)$$

In the above equations (22) and (23), the same notation designates the same parameters as in the previous equations (20) and (21).

In an analogous manner to an operation carried out by scanning the image of the object slit 211 in rotation, in the case of scanning in translation the values of the displacement increment in the direction perpendicular to the alignment direction of the axis O″z of the object slit 211 and the irradiation times satisfy similar equations.

Consequently, in the figure 4a embodiment, the device in accordance with the invention comprises calculation means denoted 8 for calculating the translation displacement increment Δu in the direction OY or OX for an orientation $\underline{u}$ in the direction OX, OY, the increment for a given object slit satisfying the equation:

$$\Delta u = Emax \frac{\overline{a}(e)}{A^u_0} \tag{24}$$

In this equation the parameters $\overline{a}(e)$ and $A^u_0$ correspond of course to the definitions given previously in this description.

Also, in the embodiment shown in figure 4a, the device in accordance with the invention also comprises means 8 for calculating the number of laser emission pulses denoted $NI_2$ and the number of translation displacements increments $\Delta u$ in the direction OY, OX. The number $NI_2$ of pulses satisfies the equation:

$$NI_2 = \frac{2\pi}{\Delta u} = ND_2 \frac{A^u_0}{\overline{a}(e)} \tag{25}$$

In this equation $ND_2$ represents the number of totally separate or adjacent images that can be formed on the cornea.

In the same way as in the case of treatment or correction by an object slit or object slit image performing a rotating scan, in the figure 4a embodiment the calculation means 8 may also be used to calculate the minimum total radiation time denoted $T_{2min}$. This satisfies the equation:

$$T_{2min} = NI_2 \frac{\tau(e)}{ND_2} = \tau(e) \frac{A^u_0}{\overline{a}(e)} \tag{26}$$

In this equation, $\tau(e)$ represents the minimum time interval between two successive irradiations of the same point on the cornea.

As will be noted from figures 3b, 3c, 3d, 3e, 4b, 4c and 4d in particular, the object slits 211, whether used during an operation to effect scanning in rotation or in translation of the area of the cornea to be treated, are symmetrical with respect to their longitudinal axis O'z or O"z. This corresponds to a particularly advantageous, non-limiting embodiment in which, without departing from the scope of the present invention, the slits may be asymmetrical with respect to the longitudinal axis O'z or O"z provided that the corresponding width of the slit at a given point z is substantially the same.

As will be noted in figure 4c, in the case of an object slit 211 used for treatment of hypermetropia by scanning in translation the object slits, whether they generate one or more lobes of the treatment laser beam FLT scanned in rotation or in translation, may advantageously comprise a curvilinear shape edge denoted C at the end. This edge at the end is, as shown to a larger scale in figure 4c, symmetrical with respect to the longitudinal axis O"z. The curvilinear shape departs from the variation law $\rho$ = constant, representing a circular arc in polar coordinates, to eliminate edge effects from the resulting profile of the total ablation obtained.

As will be noted in figure 4c, in a non-limiting way, the curvilinear shape C may be concave and convex with a point of inflection. Likewise, provided that the curvilinear shape C departs from the variation law $\rho$ = constant, the edge at the end may equally well be continuously concave, as shown in dashed outline in the enlarged view of figure 4c.

A curvilinear character of this kind for the edge of the slits at the end improves the continuity of the curvature in transitions between corrected and uncorrected areas. Thus any slit of which an edge at the end has a non-zero width or aperture could comprise the aforementioned curvilinear slit C. The curvilinear shape C, in the absence of any point of inflection, provides for transitions between corrected and uncorrected areas at which there is a discontinuity in the curvature.

Of course, in an analogous way to the embodiment of the object slits in figure 3e in the case of rotational scanning for a plurality of object slits 211 on the same diaphragm 21, in the case of treatment by scanning in translation it is also possible to use a plurality of object slits 211 on the same diaphragm. A diaphragm of this kind is shown in figure 4d, in which three slits $211_1$, $211_2$ and $211_3$ have been shown by way of non-limiting example. The various object slits are spaced in a direction perpendicular to their longitudinal axis O″z by a distance at least equal to the widest aperture $E_{max}$ thereof.

A prototype of the device in accordance with the invention was manufactured with the object slits 211 as described previously with reference to figures 3b, 3c, 3d, 4b, 4c and 4d.

To give a non-limiting example, in the case of an object slit such as that shown in figure 3b, the object slit 211 had a length substantially equal to 3.2 mm, its length being measured along the longitudinal axis O′z, and a width or maximal dimension in the direction perpendicular to the aforementioned longitudinal axis substantially equal to 0.8 mm.

An object slit 211 as shown in figure 3c had a length substantially equal to 3.2 mm and a maximal width in the order of 1.4 mm.

In the case of an object slit 211 as shown in figure 4b the length of the slit along the longitudinal axis O″z was in the order of 6 mm and its maximal width in the order of 1 mm.

Of course, the foregoing dimensions of the object slits 211 are given by way of non-limiting example only, since it is to be understood that these dimensions vary according to the total magnification of the optical system of the device in accordance with the invention. The latter may of course and advantageously be provided with an optical system offering variable magnification so that from a particular design of object slit the practitioner is in a position to choose the final dimension of the image of the lobe or lobes of the treatment laser beam FLT given by the aforementioned object slits.

In accordance with another advantageous characteristic of the device in accordance with the invention, with particular reference to the figure 4a embodiment in which the diaphragm 21 is fixed, each slit may advantageously have a variable profile to provide for compensation for any irregular distribution of the light energy over the cross-section of a lobe of the treatment laser beam FLT.

As will be noted in figure 4e, the variable slit 211 may comprise at least one edge made up of mobile strips denoted 2110, these strips being movable in translation in a direction perpendicular to the longitudinal axis O″z of the slit. The mobile strips 2110 may of course be disposed to slide relative to each other, each being adapted to be driven by the intermediary of a motor or like means 2111. It will be understood of course that in the case of the figure 4e embodiment the dimensions of the object slit 211 may be increased to facilitate implementation of the movable strips, the magnification of the optical system of the device in accordance with the invention being adjusted accordingly.

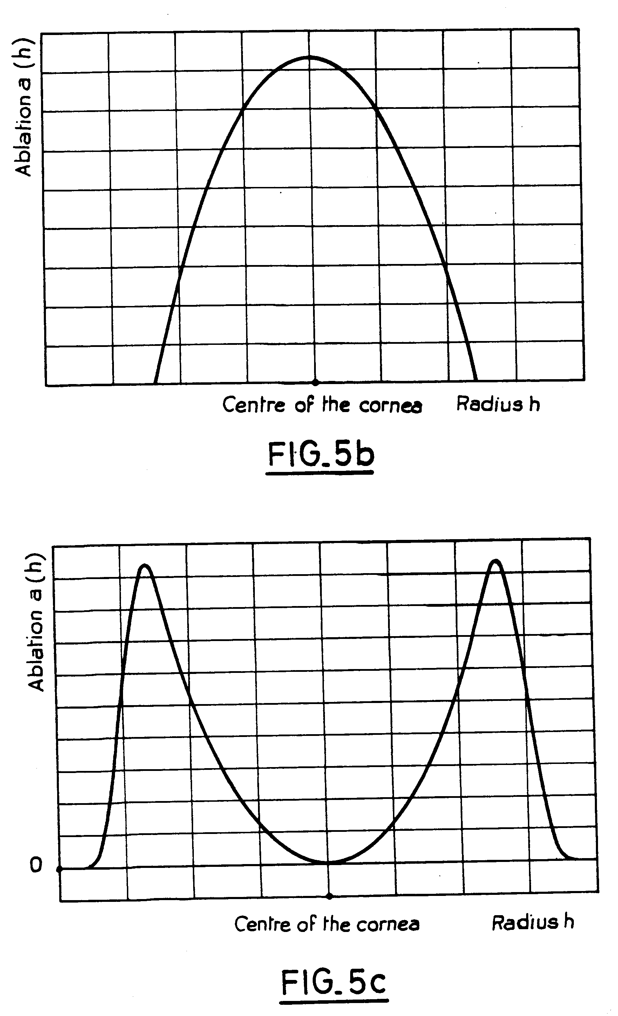

One example of an operation for treatment of myopic astigmatism by keratomileusis using the device in accordance with the invention shown in figure 4a and scanning of the area to be treated in translation will now be described with reference to figure 5a.

The total resulting ablation is in this instance obtained by means of a slit such as that shown in figure 4d, for example, the image of the slit or the lobe of the treatment laser beam FLT being displaced in a direction perpendicular to the longitudinal axis O″z in consecutive elementary increments. The elementary displacement increments being equal, the effect of the treatment is to produce a channel of uniform parabolic profile. The length of the channel is of course equal to the distance over which the slit is displaced and its width is equal to the length of the slit.

In a particularly advantageous method of working, two operations are effected along two perpendicular axes to achieve complete correction of the cornea COR.

In the case of myopia, this method of working has the following advantages:
- it eliminates the problem of precisely focussing the end or the image of the slit on the rotation axis in the case of scanning in rotation, and
- it enables all types of astigmatism to be corrected.

The longitudinal axis O″z of the slit being oriented in the direction OX, for example, in figure 2a, irradiation of the object slit 211 in successive positions spaced by a constant translation increment $\Delta Y$ in the direction OY in figure 2a within a range of displacement ranging between $-R/\sqrt{2}$ and $+R/\sqrt{2}$ serves to obtain with respect to the axis OX an ablation profile B(X) defined by the equation:

$$B(X) = \bar{a}(e) \left[ \frac{E(X)}{\Delta^x_y} \right], \quad \forall X \in \left[ \frac{-R}{\sqrt{2}}, \frac{R}{\sqrt{2}} \right] \qquad (27)$$

In this equation:
- E(X) represents, of course, the profile of the slit at the abscissa X and $\Delta^x_y$ represents the constant translation displacement increment in the direction Y, the slit being oriented in the direction X,
- R is the radius of the area to be corrected centered at O″.

15

As previously mentioned in this description, when the axes OX and OY from figure 2a correspond to the principal directions of the meridians corresponding to the ends of the curves at the centre of the cornea, the principal astigmatism directions, the ablation profile to be obtained is expressed by the equation:

$$A(X,Y) = A^x_0 \left( \frac{1}{2} - \frac{X^2}{R^2} \right) + A^y_0 \left( \frac{1}{2} - \frac{Y^2}{R^2} \right) \qquad (28)$$

In equation (28) the parameters $A^x_0$ and $A^y_0$ satisfies the equations:

$$A^x_0 = \frac{R^2}{2} \left( \frac{1}{r_x} - \frac{1}{r} \right) \qquad (29)$$

$$A^y_0 = \frac{R^2}{2} \left( \frac{1}{r_y} - \frac{1}{r} \right) \qquad (30)$$

The ablation function may be regarded as the result of summing two ablation functions, one a function of X only and the other a function of Y only. In equations (29) and (30), $r_x$ represents the radius of curvature of the cornea in the direction OX and $r_y$ represents the radius of curvature in the direction OY, r representing the radius of curvature of the cornea in a meridian direction at the azimuth angle $\beta$ previously mentioned.

Adopting the following notation:

$$A_0 = \frac{A^x_0 + A^y_0}{2} \qquad (31)$$

$$R_X = R \sqrt{\frac{A_0}{A^x_0}} \qquad (32)$$

$$R_y = R \sqrt{\frac{A_0}{A^y_0}} \qquad (33)$$

16

the equation for the resulting total ablation function may be written:

$$A(X,Y) = A_0 \left( 1 - \frac{X^2}{R_x^2} - \frac{Y^2}{R_y^2} \right) \qquad (34)$$

The iso-ablation curves are therefore ellipses in the general case and the equation for the ellipse which delimits the ablation contour is:

$$\frac{X^2}{R_x^2} + \frac{Y^2}{R_y^2} = 1 \qquad (35)$$

As shown in figure 5a, theory indicates that the resultant ablation should extend from $-R_X$ to $+R_X$ on the OX axis and from $-R_Y$ to $+R_Y$ on the OY axis. The ablation profile is thus contained within two orthogonal rectangles with respective lengths $2R_X$ and $2R_Y$ and the same width $R\sqrt{2}$ and whose common area is square inscribed in the circle Ce of radius R centered at O. The ablation profile obtained is perfect within the square where they intersect although a satisfactory approximation of the ablation profile is nevertheless obtained outside the square in the areas peripheral to the latter, the areas FGHI in figure 5a, the central area consisting of the square being denoted A.

In the case of pure myopia with no astigmatism, $r_x = r_y$ and $A_0 = A^x_0 = A^Y_0$.

Thus correction or treatment by means of an object slit scanned in translation along two orthogonal directions produces an optimal effect where the areas scanned by the treatment laser beam FLT in the aforementioned directions intersect, that is over a square in plane projection.

To extend this action beyond the intersection square and to obtain satisfactory correction over a substantialy circular area it is possible to extend the lateral scanning of the treatment laser beam FLT while modulating the displacement increment $\Delta u$ between two adjacent positions, the aforementioned increment $\Delta u$ remaining constant in the intersection area, of course.

It has been shown that the ablation profile in the first area made up of the three areas A, F and H (that is for $-R/\sqrt{2} \leq X \leq +R/\sqrt{2}$) is achieved by irradiating a slit parallel to the axis OX and moving by increments $\Delta^Y_X$ in the direction perpendicular to the OY axis.

Likewise, the ablation profile in the area made up of the area I, A and G (that is for $-R/\sqrt{2} \leq Y \leq R/\sqrt{2}$) is achieved by irradiating a slit parallel to the OY axis moved in increments $\Delta^Y_X$ or $\Delta u$ along the OX axis.

This second operation, correction of the profile along OY, does not modify the profile along an axis parallel to OX, but deepens it uniformly (Y = constant) in particular by an amount $A^Y_0$ over all of the axis OX, that is for Y = 0.

To complete the resulting total ablation profile along OX and to avoid any discontinuity for $X = \pm R/\sqrt{2}$ the scanning in translation along OX of the slit which generates the ablation profile along OY can be extended beyond these values, with the translation displacement increment along OX increasing with X for $|X| > R/\sqrt{2}$.

The device in accordance with the invention as shown in figure 4a uses the calculation means 8 to determine the value of the linear displacement increment denoted $\Delta^Y_X$, for example to obtain an exact extension of the parabolic profile for Y = 0, the translation displacement increment for a corresponding ablation function satisfying the equation:

$$A(X,0) = A_0\left(1 - \frac{X^2}{R_x^2}\right) \qquad (36)$$

The translation displacement increment then satisfies over all of the treatment domain comprising areas F, A and H in figure 5a the equation:

$$\Delta^Y_X(X) = \begin{bmatrix} E_{max} \dfrac{\overline{a}(e)}{A_0\left(1- \dfrac{X^2}{R_x^2}\right)}, & \text{for } \dfrac{R}{\sqrt{2}} \leqslant |X| \leqslant R_x \\[4em] E_{max} \dfrac{2\,\overline{a}(e)}{A^Y_0}, & \text{for } |X| < \dfrac{R}{\sqrt{2}} \end{bmatrix} \qquad (37)$$

In equations (36) and (37), $R_X$ defines the total irradiation domain in the X direction.

The resulting total ablation function $A_X(X,Y)$ which defines the resulting total ablation in rectangles F and H in figure 5a, that is to say for

$$\frac{R}{\sqrt{2}} \leqslant |X| \leqslant R_X, \; |Y| \leqslant \frac{R}{\sqrt{2}},$$

satisfies the equation:

$$A_X(X,Y) = \frac{E(Y)}{\Delta^Y_X}\,\overline{a}(e) \qquad (38)$$

In this equation E(Y) represents, of course, the profile of the slit used, the slit having its longitudinal axis O″z oriented in the Y direction and $\Delta^Y_X$ corresponding to the values of equation (37) for the values of X included in the areas F and H.

The working method previously described with a slit procuring scanning of the treatment laser beam FLT in translation or using a slit with a parabolic profile as explained previously in this description thus yields an ablation profile which over the periphery of the area of an ellipse denoted E in figure 5a, with half-axes $R_x$ and $R_y$, contains eight "perfect" points by which is meant points of zero ablation.

Of course, in the case where there is a requirement not to irradiate the cornea COR beyond an area of radius R it is possible to mask the latter with a mask comprising a circular hole of radius R.

There are shown in figures 5b and 5c respectively a profile characteristic of keratomileusis ablation for myopia with no astigmatism and a profile characteristic of keratomileusis ablation for hypermetropia.

18

In figures 5b and 5c units have not been marked on the coordinate axes. In the case of an operation by keratomileusis on myopia, an ablation corresponding to a correction of 15 diopters has a depth of 0.15 mm and extends over an area 5 mm in diameter. The initial radius of curvature is increased to 10.6 mm.

In the case of figure 5c, in which the units have not been shown on the coordinate axes, an ablation corresponding to a correction of 15 diopters has a depth of 0.15 mm and extends over an area 9 mm in diameter. The initial radius of curvature of 7.8 mm is reduced to 5 mm.

The device in accordance with the invention makes it possible to overcome the limitations of prior art devices through the use of an illumination and treatment laser beam the specific shape and displacement of which are computed so that their combination produces the required ablation shape.

When the slit or slits is or are irradiated by a particular pulse from the laser the image of the slit(s) projected onto the cornea COR is, so to speak, etched on to the surface and causes by photodecomposition the elementary ablation in question. The sum of these elementary ablations distributed over the cornea in accordance with the mathematical laws previously established produces the required modification to the shape of the cornea.

Unlike the prior art devices, in which the concepts of illumination time were involved, the concepts of the laser pulse frequency and of the speed of displacement of the object slit (or its image) are replaced by the concepts of linear or angular increments, as appropriate, between two adjacent positions of the image or of the lobe of the treatment laser beam. Here "adjacent" is to be understood in the geometrical rather the temporal sense. In other words, the fact that two geometrically adjacent, that is to say geometrically consecutive, elementary ablations are temporally consecutive is not relevant. Generally speaking, they are not.

All the considerations previously mentioned combined with the concept of a threshold relating to each elementary ablation serves through summation of the elementary ablations in question to obtain a corrected or treated surface that is particularly satisfactory and the degree of roughness of which is substantially less than 1 $\mu$m.

In the case of rotational scanning, there is generally projected onto the eye OE a beam whose transverse cross-section is caused to rotate about the projection axis O, which is of course substantially coincident with the optical axis of the eye to be treated. The cross-section of the treatment laser beam FLT is of elongate shape, of course, and in a particularly advantageous way has at least one or several lobes as defined previously. The generatrix at the end of the treatment laser beam or the corresponding lobe coincides with the rotation axis O in figure 2a. The ablation is done by applying the beam to a large number of successive angular positions, spaced by the appropriate angular increment of rotation about the axis O. To obtain the required correction the cross-section of the treatment laser beam FLT, the energy density per unit surface area of which is substantially constant, has the profile as defined previously on the basis of the object slits 211.

In the second embodiment, in particular using the device as shown in figure 4a, the resulting total ablation is obtained by scanning the treatment laser beam FLT in translation by successive linear increments. The displacement takes place in the direction perpendicular to the longitudinal dimension of the largest dimension of the lobe of the laser beam FLT and perpendicular to the optical axis O of the eye OE. Several operations are needed to carry out a complete treatment.

Of course, and in a non-limiting way, it is possible to carry out several operations, for example, the treatment laser beam FLT undergoing after each pass a rotation of a fraction of a circle about the optical axis O. After n passes (n/2 if the beam is symmetrical), the combination of the aforementioned operations produces an nth order circular symmetry ablation more or less approximating the required effect.

A particularly advantageous instance, as previously described, is the use of a beam of parabolic cross-section the lobes of which have a parabolic shape as described previously, the laser beam being scanned in two passes along two perpendicular directions.

Compared with rotational scanning of the treatment laser beam FLT, scanning in translation for correction of myopia avoids a problem specific to rotary scanned beams, namely that the centre of the eye where the ablation is strongest coincides with the centre of rotation and that the latter is situated by design at an end of the impact area. In the event of any error in aligning this impact area with respect to the rotation axis, total absence of ablation (or its opposite, excessive ablation) may result in the immediate vicinity of the centre of the cornea. This problem is absent in the case of beams scanned in translation.

Furthermore, in the case of scanning in translation the choice of this scanning mode (along two orthogonal directions) provides a simple means of correcting astigmatism. For this, it is sufficient for the two orientations of the beam along the directions OX and OY to coincide with the principal directions of astigmatism. It then suffices to change the average density of exposure by changing the length of the linear increments between the two orthogonal passes to obtain an ablation of elliptical rather than circular symmetry.

The translational scanning treatment laser beams may of course be used in various ways, the beams with different orientations being applied either successively or simultaneously.

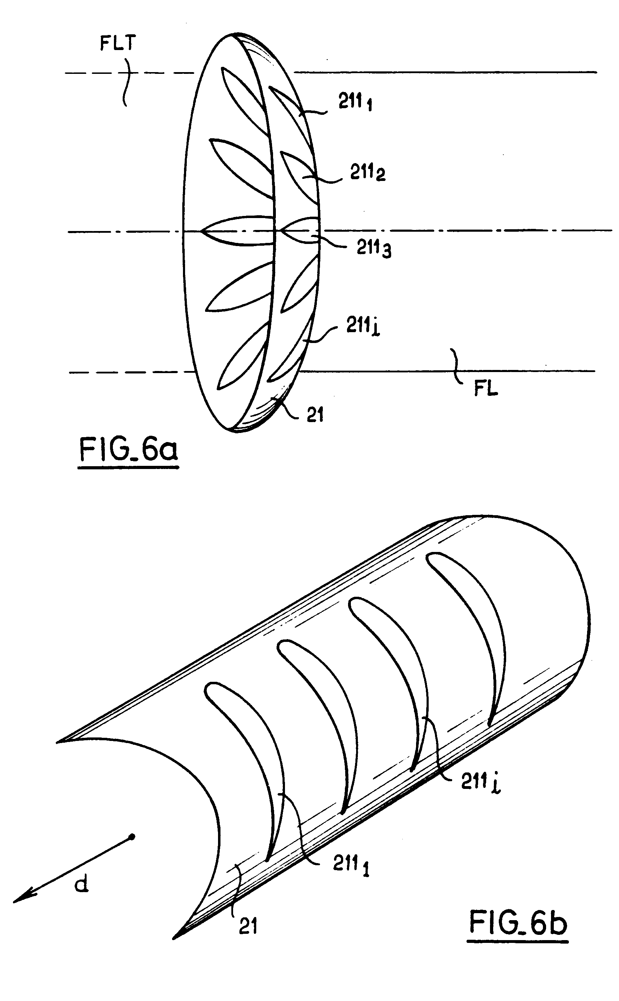

Another particularly advantageous embodiment of an object slit 211 and a diaphragm 21 will be described with reference to figures 6a through 6d.

Referring to figure 6a and figure 6b, the object slits as previously shown in figures 3b, 3c, 3d, 3e, 3f, 4b, 4c and 4d may advantageously be formed on a diaphragm 21 with a curved surface matching the surface of the cornea COR. This embodiment improves the quality of focussing of the image of the object slit on the cornea COR. In the case of figure 6a, the curved surface forming the diaphragm 21 is a spherical dome and the diaphragm may be rotated about its axis of symmetry, as previously described. In the case of object slits scanned in translation, the curved surface forming the diaphragm 21 may advantageously, and as shown in figure 6b, be a semicylindrical surface the longitudinal axis of which is oriented in the translation direction d, the object slits having their axis O″z perpendicular to the longitudinal axis of the aforementioned half-cylinder.

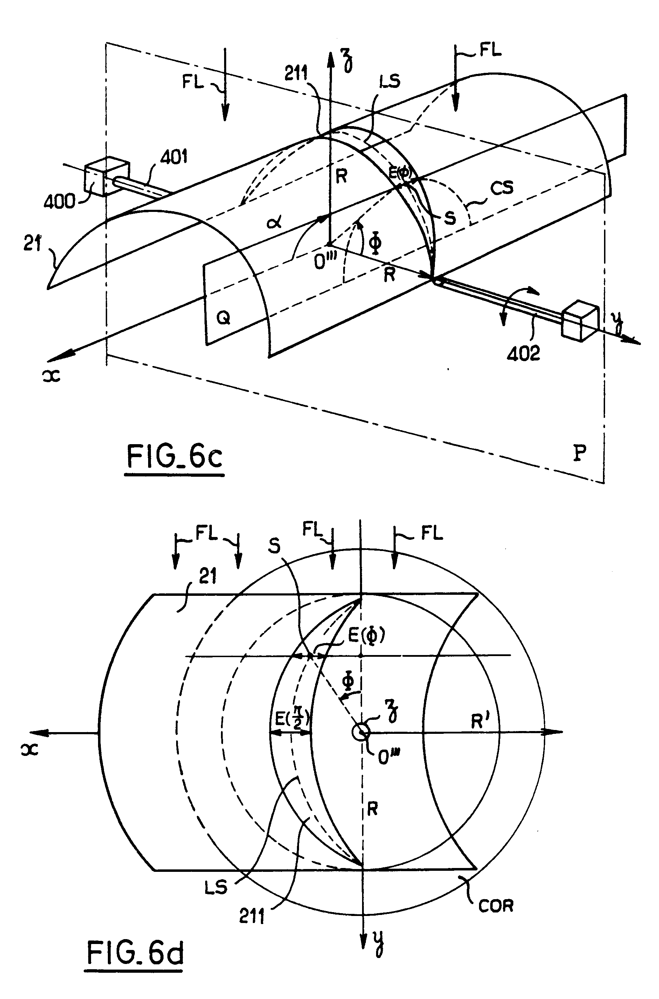

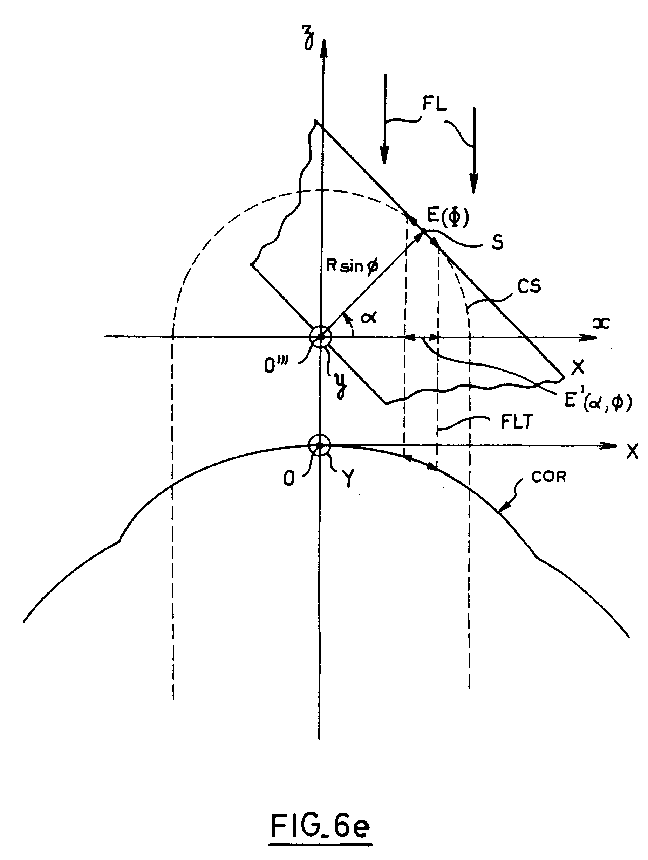

A particularly advantageous embodiment of the diaphragm 21 will be described with reference to figures 6c, 6d and 6e.

In the aforementioned figure 6c the diaphragm 21 comprises a semicylindrical surface of radius R with a longitudinal axis O‴x. The semi-cylindrical surface has an object slit 211 with an aperture or width in the direction O‴x denoted $E(\phi)$. The aperture is, for example, symmetrical to a plane P orthogonal to the longitudinal axis O‴x, this plane containing the directions O‴y and O‴z orthogonal to the direction of the longitudinal axis O‴x. In figure 6c, S represents the middle of the aperture or the width of the slit at a height z corresponding to a given angle $\phi$, the angle $\phi$ being defined as the angle between the radius vector O‴S of a point S on the geometrical locus LS, the curve of symmetry of the object slit 211, and the direction 0y. The width $E(\phi)$ of the object slit 211 satisfies the equation:

$$E(\phi) = E(\tfrac{\pi}{2})\sin^3\phi \qquad (39)$$

In this equation, $E(\pi/2)$ represents the maximum width or aperture of the object slit 211 for $\phi = \pi/2$.

It will of course be noted, as will be described in more detail later, that in the case of an operation by keratomileusis to cure myopic astigmatism the radius R of the semicylindrical surface constituting the diaphragm 21 determines the area within which the practitioner operates on the cornea COR. To give a non-limiting example, the aforementioned radius is taken as equal to the operating area, the magnification of the focussing optics being taken as equal to unity. It is obvious that any semicylindrical diaphragm of appropriately similar shape could be used, the magnification of the focussing optics being adapted accordingly.

To correct the cornea COR by keratomileusis for myopic astigmatism, for example, the diaphragm 21 as shown in figure 6c is disposed relative to the cornea COR so that its concave side faces towards the area of the latter to be treated. The cornea COR is assumed to have a circular surface of radius R′ and the object slit 211 as shown in figure 6c is illuminated by the laser beam FL. The longitudinal axis O‴x and the transverse axis P‴y of the diaphragm are oriented in the principal directions of astigmatism OX, OY of the cornea COR, these principal directions having been determined beforehand by the practitioner.

The device in accordance with the invention further comprises drive means 400 for rotating the diaphragm 21 about the axis O‴y, the rotation drive means 400 advantageously comprising a stepper motor and two rotation half-shafts 401, 402 driven by the latter.

As will be noted on observing figures 6c, 6d and 6e, the width $E(\phi)$ of the object slit 211 which is also shown in figure 6c by the intersection of the slit and a plane Q for which the equation is $Y = R \cos\phi$, is projected on the axis OX as an image of width $E(\alpha,\phi)$ when the slit 211 is illuminated with parallel light, for example. The width of the slit projected on the axis OX satisfies the equation:

$$E'(\alpha,\phi) = E(\phi). \sin\alpha \qquad (40)$$

In this equation, $\alpha$ represents the angle of inclination by which the diaphragm 21 is rotated and in particular of the axis O‴z thereof relative to the direction OX. The slit 211 turns substantially on a sphere with the same radius as the cylinder and, as the diaphragm 21 is rotated, any middle point S at a height z corresponding to a given angle $\phi$ performs a circle CS in the aforementioned plane Q, as shown in figures 6c and 6e.

If it is assumed that the ablation function $A(\alpha,\phi)$ corresponds to the ablation function $A(X,Y)$ defined by equation (34) and is proportional to the number of pulses received for an elementary displacement less than

E'($\alpha,\phi$) and therefore less than the width E'($\alpha,\phi$) of the image of the slit on the axis OX divided by the elementary displacement $\Delta$x($\alpha$) (along the OX axis) for each laser pulse, we may write:

X($\alpha$) = Rsin$\phi$ Cos$\alpha$ and $\frac{\delta X}{\delta\alpha}$ . d$\alpha$ = $\frac{\delta}{\delta\alpha}$ Rsin$\phi$Cos$\alpha$d$\alpha$ = - Rsin$\phi$ Sin$\alpha$ d$\alpha$

and $\Delta$X($\alpha$) = R sin $\phi$ sin$\alpha\Delta\alpha$

whence

$$A(X,Y) = A(\alpha,\phi) = \frac{E'(\alpha,\phi)}{\Delta X(\alpha)}$$

that is

$$A(\alpha,\phi) = \frac{(E)\phi}{R\ Sin\phi\Delta\alpha} \tag{41}$$

Given the chosen ablation function A(X,Y) defined by equation (34) above, OX and OY are chosen such that Rx $\leqq$ Ry and R is chosen such that R = Ry as shown in figures 6d and 6e in particular.

Using the same notation as previously, the ablation function may be written:

$$A(X,Y) = \frac{A0}{2}\left(1 - \frac{X^2}{R_x^2} - \frac{Y^2}{R^2}\right) \tag{42}$$

Given the equations:

X = R sin$\phi$ cos$\alpha$

Y = R cos$\alpha$

the ablation function becomes:

$$A(X,Y) = A(\alpha,\phi) = \frac{A0}{2}\left(1 - \frac{R^2}{R_x^2}\cos^2\alpha\right)\sin^2\phi \tag{43}$$

Given equations (41) and (43) above, the ablation function may be related to the law of the aperture of the slit E($\phi$) and the rotation increment $\Delta$ $\alpha$ by the equation:

$$\frac{E(\phi)}{\Delta\alpha} = \frac{RA0}{2} \cdot \sin^3\phi \cdot \left(1 - \frac{R^{x}}{R_x^2}\cos^2\alpha\right) \tag{44}$$

It then suffices to choose:

E($\phi$) = E ($\frac{\pi}{2}$) sin$^3\phi$

and

$$\Delta\alpha = \Delta 0 (1 - \frac{R^2}{Rx^2} \cos^2 \alpha)^{-1}$$

Choosing (E($\pi$/2) and $\Delta$ 0 for a given semicylindrical diaphragm produces the required profile.

By modulating the angular rotation increment $\Delta \alpha$, the previously described embodiment can correct astigmatism of the cornea and myopia without any problems of edge discontinuities at the periphery of the correction area or excessive ablation at the centre of the cornea. The junction between the corrected area and the uncorrected area is perfect. Also, in the absence of any astigmatism the previous equations hold, given that Rx = Ry = R. In all cases, the X and Y ablation functions depend only on $\alpha$ and $\phi$ respectively.

To facilitate use of the device in accordance with the invention as shown in figure 3a or in figure 4a the calculation means 8 may comprise a microcomputer 80 with its peripheral devices. The memory areas of the microcomputer store programs and/or subroutines for calculating the numbers of laser pulses $NI_1$, $NI_2$ previously mentioned in the description, the total irradiation times $T_{1min}$, $T_{2min}$, and sub-routines for sequencing and synchronising the displacement of the treatment laser beam FLT. These sequencing programs are used, for example, to generate rotation or translation displacement commands scdr and scdt and laser emission commands sce. The program or subroutine can also include a program for modulating the rotation increment $\Gamma$ as a function of the azimuth angle $\beta$ or the translation increment $\Delta u$ as a function of the value of the X or Y ascissa of the rotation increment $\Delta \alpha$.

To facilitate the work of the practitioner the microcomputer 80 may further comprise in its memory area a "menu" type program inviting the practitioner, through an interactive type dialogue, to define at least the principal directions of astigmatism of the eyeball relative to a reference marker, the principal directions having been established by the practitioner as a result of a diagnosis.

The "menu" program may advantageously also invite the practitioner to specify the value of the parameter R defining the optical area for operation and correction of the cornea COR. It may also invite the practitioner to designate the treatment method i.e. scanning the object slits or images of the object slits in rotation or in translation. Finally, the type of operation may be specified according to the particular case under treatment.

The microcomputer 80 can of course be connected by a BUS type link to the means 5 for synchronising the displacement of the image of the lobe or lobes of the treatment laser beam FLT. The means 5 for synchronising the displacement of the image may advantageously comprise an input/output interface circuit generating from rotation or translation displacement commands scdr and scdt and emission commands sce respective commands SCDR, SCDT, SCE for the displacement control means 4 and the laser emission means 1. The input/output interface circuit will not be described in detail, as it may be provided by any conventional type interface with provision for controlling the stepper motor in particular.

Finally, to facilitate the work of the practitioner, following his diagnosis, the device in accordance with the invention may comprise a set of diaphragms each comprising an object slit 211 as defined and described with reference to figures 3b, 3c, 3d, 3e, 3f, 4b, 4c, 4d, 4e, 6a, 6b, and 6c.

There has thus been described a device for performing surgery on the cornea in which rotational or translational scanning of a laser beam having at least one lobe of elongate cross-section produces a precise law of ablation over the area of the cornea COR of the eye to be corrected. Laboratory tests have shown that, compared with prior art devices in which the depth of ablation was controlled by the time of exposure to the treatment laser beam, the corrected surfaces after treatment, that is to say the surfaces of the cornea serving as the input optical surface of the eye of the patient, show a much reduced degree of roughness, thus conferring superior optical qualities on the surfaces of the treated cornea. It has been observed that the degree of roughness of the surfaces after treatment does not exceed 1 $\mu$m. The degree of roughness of the corneal surfaces after treatment with the prior art devices may be explained by the fact that these devices have the disadvantage of applying the laser emission power simultaneously to the major part of the cornea, the effect of which is to create an acoustic shock wave resulting from simultaneous vapourising of material over the anterior surface of the cornea. This kind of phenomenon can also have unwanted physiological consequences, such as ejection of endothelium cells, for example. The device in accordance with the invention makes it possible to eliminate the disadvantages of these devices since the resulting total ablation when the device in accordance with the invention is used results from the summation of elementary ablations distributed over the cornea according to precise mathematical laws, each elementary ablation being carried out with minimal energy density.

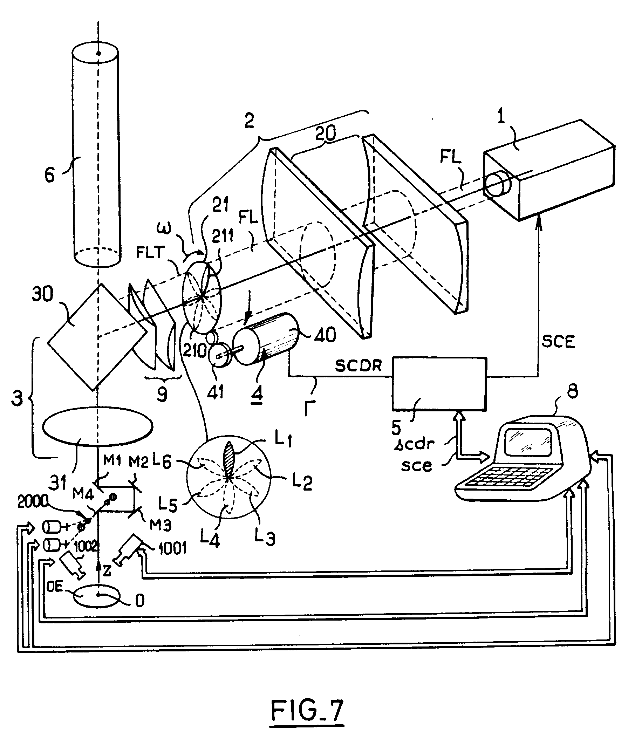

Of course, the device in accordance with the invention is not limited to refractive eye surgery. It may also constitute a device for shaping or correcting the shape of an object by laser treatment of the surface of the object. In this case, the device comprises the means 2 for generating a treatment laser beam FLT comprising at least one lobe L1 ... L6 of elongate cross-section and means 3 for focussing the image of the lobe or lobes of the treatment laser beam FLT onto the area of the object OE to be corrected. The means 4 for moving the image of the lobe or lobes of the treatment laser beam FLT over the area of the object to be corrected serve to move the latter over the area of the object to be corrected. The means 5 for synchronising movement of the image of the lobe or lobes of the treatment laser beam FLT over the area of the object OE to be corrected with the treatment laser beam pulses serve to perform the correction or shaping by summing a plurality of elementary discrete ablations. As shown in figure 7, the image of the lobe or lobes of the laser beam is focussed in such a way that the generatrix of one end of the lobe or lobes or the longitudinal axis of symmetry of a lobe or the lobes of the laser beam FLT is coincident with the axis of symmetry OZ of the object to be treated or of an elementary surface of the object to be treated. The means 4 for moving the image of the lobe or lobes of the treatment laser beam FLT over the area of the object to be corrected serve to move the image of the lobe or lobes L1 ... L6 of the laser beam in rotation about the end generatrix or the longitudinal axis of symmetry of the lobes of the treatment laser beam FLT. The rotation is applied in rotation angle increments. The device corresponds substantially to the embodiment of figure 3a.

Furthermore, in an embodiment corresponding to that of figure 4a of a device for shaping or correcting the shape of an object by laser treatment, the means 4 for moving the image of the lobe or lobes of the treatment laser beam FLT over the area of the object to be treated provide for movement in translation in a direction d substantially perpendicular to the largest dimension 0z of the lobe of the treatment laser beam FLT. The movement in translation may be effected in displacement increments $\Delta u$, the movement in translation being defined by u = X or u = Y defining a plane tangential to the surface of the object OE at the point 0 on the axis of symmetry of the object or an elementary area of the latter to be treated.

A non-limiting alternative embodiment of the device in accordance with the invention for shaping or correcting the shape of an object or for performing refractive eye surgery will be described with reference to figure 7, this embodiment being based on the embodiment of figure 3a or figure 4a. Referring to figure 7, the device in accordance with the invention further comprises a real time shape recognition system comprising at least two video cameras 1001, 1002 viewing the object or the eye OE to be treated and transmitting image data to the calculation means 8. The video cameras 1001, 1002 allow for monitoring the progress of the shaping or correction of the object during the treatment process. The shape recognition means may comprise shape recognition means available through normal trade channels and will not be described in detail.

As shown in figure 7, a series of mirrors M1, M2, M3, M4 deflect the treatment laser beam FLT. At least one of these mirrors, the mirror M4, is mounted on a gimbal 2000. The two frames of the gimbal mounting are shown in cross-section in figure 7 to avoid overcomplicating the drawing. Drive and orientation adjustment means for the adjustment mirror comprise DC or stepper motors, for example. These motors are controlled by the shape recognition means 1001, 1002 through the intermediary of the calculating means 8, using a bus type link. The shape recognition system therefore serves to monitor the progress of the correction or treatment during the process and to control the deflection of the treatment laser beam FLT by means of the mirror M4 in the event of uncontrolled movement of the object or of the eye of the patient. In the latter case the practitioner can advantageously make coloured marks on the cornea of the patient before the treatment begins, using methylene blue, for example. Note, however, that if an arrangement of this kind is used with the device in accordance with the invention as shown in figure 4a, the shape recognition means 1001, 1002 can control the focussing lens 430 directly by means of an X-Y movable table 43.

The device in accordance with the invention is therefore usable for shaping or correcting the shape of mechanical objects such as contact lenses or intra-ocular implants and for refractive eye surgery.

## Claims

1. Device for shaping the shape of an objects (OE) laser ablation of a surface of said object (OE) according to an ablation function, said device comprising:
   . means (1) for generating a pulsed laser beam (FL) having pulses and an energy density,
   . slit means (21) having at least one slit (21) intercepting said laser beam (FL), said at least one slit (211) having a profile function proportional to said ablation function,
   . means (3) for forming an image of said at least one slit (211) onto an area of said surface of said object (OE),

23

- means (4) for displacing said image of said at least one (211) over said area by steps of a given increment, corresponding to elementary discrete ablations of said surface of said object (OE),
- means (5) for synchronizing said increment, said pulses and said energy density, so that the total ablation resulting from the summation of said elementary discrete ablations meets said ablation function.

2. Device according to claim 1, wherein said at least one slit (211) has an adjustable profile function to enable compensation of irregular distribution of said energy density in the cross section of intercepted portion of said laser beam (FL).

3. Device according to claim 2, wherein said at least one slit (211) comprises at least one edge made up of strips (2110) mobile in translation, in a direction perpendicular to the longitudinal axis of said slit (211).

4. Device according to claim 1, wherein said ablation function being defined by a function A(h) having a symmetry of revolution about an axis (Oz) perpendicular to said surface of said object (OE), h being the distance to said axis (Oz), said profile function of said at least one slit (211) in a polar ($\rho,\theta$) coordinate system is given by

$$\theta(\rho) = \frac{\Gamma}{\bar{a}(e)} A(\rho)$$

in which equation :

$\Gamma$     is an angular rotation increment,

$\bar{a}(e)$     represents the average thickness removed by irradiation of each laser pulse.

5. Device according to claim 4, wherein an anisotropic ablation function $A(h,\beta)$ is obtained by modulating said angular rotation increment $\Gamma$ as a function of azimuth angle $\beta$ about said axis (Oz):

$$A(h,\beta) = \frac{\bar{a}(e)}{\Gamma(\beta)} \theta(h)$$

6. Device according to claim 4, further comprising means (8) for calculating the angular rotation increment $\Gamma$ which satisfies the equation :

$$\Gamma = \theta_{max} \frac{\bar{a}(e)}{A_0}$$

in which equation :

$\theta_{max}$ represents the maximum aperture angle of said profile function of said at least one slit (211),

$A_0$ is the maximum value of said ablation function.

7. Device according to claim 4, further comprising means (8) for calculating the number $NI_1$ of laser pulses and the number of rotation increments $\Gamma$ , the number $NI_1$ of pulses satisfying the equation :

$$NI_1 = N_{D1} \frac{A_0}{\bar{a}(e)}$$

in which equation :

24

$ND_I$ represents the number of totally separate or adjacent images of said at least one slit (211),

$A_o$ is the maximum value of said ablation function.

8. Device according to claim 7, further comprising means (8) for calculating the minimum total irradiation time $T_{1min}$ which satisfies the equation:

$$T_{1min} = N_{I1} \frac{\tau (e)}{N_{D1}} = \tau (e) \frac{A_0}{\bar{a}(e)}$$

in which equation:

$\tau$ (e) represents the minimum time interval between two successive irradiations of a same point of said area.

9. Device according to claim 4, wherein said means (4) for displacing said image of said at least one slit (211) comprise :

. a diagram (21) comprising said at least one slit (211),

. drive means for rotating said diaphragm (21), comprising a toothed ring (210) disposed at the periphery of said diaphragm (21), and a stepper motor (40) the drive shaft of which is fitted with at least one toothed wheel (41) meshing with said toothed ring (210).

10. Device according to claim 5, for refractive eye surgery, wherein said surface of said object (OE) is the external face of the cornea (COR), and wherein to compensate for astigmatism of the eye by re-establishing the symmetry of revolution of said cornea (COR), said device comprises means (8,5) for modulating said angular rotation increment $\Gamma$ as a function of said azimuth angle $\beta$ =

$$\Gamma ( \beta ) = \theta_{max} \frac{\bar{a}(e)}{A_o(\beta)}$$

is which equation :

$\theta_{max}$ represents the maximum aperture angle of said profile of said at least one slit (211),

$A_o(\beta)$ represents the maximum value of said ablation function in the direction with azimuth angle $\beta$.

11. Device according to claim 4, for refractive eye surgery, wherein said surface of said object (OE) is the external face of the cornea (COR) and wherein to compensate for astigmatism of the eye by re-establishing the symmetry of revolution of said cornea (COR), said means (3) for forming an image of said at least one slit (211) comprises an anamorphic optical system (9) the magnification of which depends on azimuth angle $\beta$ about said axis (Oz).

12. Device according to claim is, for refractive eye surgery, wherein said surface of said objects (OE) is the external face of the cornea (COR) and wherein to compensate for astigmatism of the eye by re-establishing the symmetry of revolution of said cornea (COR), said device comprises at least one auxiliary diaphragm with an object slit (211) of circular arc shape with a specific radius of curvature.

13. Device according to claim 4, for refractive eye surgery, wherein said surface of said object (OE) is the external face of the cornea (COR) and wherein, for treatment of myopia, said ablation function A(h) satisfies the equation

$$A(h) = A_o (1 - \frac{h^2}{R^2}) \quad 0 \leqslant h \leqslant R$$

25

in which equation =

A$_o$     is the maximum value of said ablation function,

R     represents the radius of said cornea (COR).

14. Device according to claim 4, for refractive eye surgery, wherein said surface of said object (OE) is the external face of the cornea (COR) and wherein, for treatment of hypermetropia, said ablation function A-(h) satisfies the equation:

$$A(h) = A_o \frac{h^2}{v^2} \quad O \leq h \leq v \quad (v < R)$$

is which equation:

A$_o$     is the maximum value of said ablation function,

R     represents the radius of said cornea (COR).

15. Device according to claim 1, wherein said ablation function being defined by a cylindrical function A$_u$-(u), u being one coordinate of a cartesian coordinate (u,v) system in a plane tangential to said surface of said object (OE), said profile function of said at least one slit (211) in a cartesian (E,z) coordinate system is given by =

$$E(z) = \frac{\Delta v}{\bar{a}_u(e)} A_u(z)$$

in which equation =

$\Delta_v$ is a translation displacement increment in the direction v.

$\bar{a}_u(e)$ represents the average thickness removed by irradiation of each laser pulse in the u direction.

16. Device according to claim 15, wherein said at least one slit (211) has an adjustable profile function to enable compensation of irregular distribution of said energy density in the cross section of intercepted portion of said laser beam (FL).

17. Device according to claim 16, wherein said at least one slit (211) comprises at least one edge made up of strips (2110) mobile in translation.

18. Device according to claim 15, further comprising means (8) for calculating the translation displacement increment $\Delta_v$ which satisfies the equation:

$$\Delta_v = E_{max} \frac{\bar{a}_u(e)}{A_u^o}$$

in which equation =

$E_{max}$ represents the maximum width of said profile function of said at least one slit (211).

$A_u^o$ is the maximum value of said ablation function.

19. Device according to claim 15, further comprising means (8) for calculating the number $NI_2^v$ of laser pulses and the number of translation displacement $\Delta_v$ in the v direction, the number $NI_2^v$ of pulses satisfying the equation:

$$NI_2^v = ND_2^v \; \frac{Au^0}{\bar{a}_u(e)}$$

in which equation:

$ND_2^v$ represents the number of totally separate or adjacent images of said at least one slit (211) in the v direction,

$A_u^0$ is the maximum value of said ablation function.

20. Device according to claim 19, further comprising means (8) for calculating the minimum total irradiation time $T_{2\,min}^v$ in the v direction which satisfies the equation:

$$T_{2\,min}^v = NI_2^v \; \frac{\tau^v(e)}{ND_2^v} = \tau^v(e) \; \frac{Au^0}{\bar{a}_u(e)}$$

in which equation:

$\tau^v(e)$ represents the minimum time interval between two successive irradiations of a same point of said area in the v direction.

21. Device according to claim 15, wherein said means (4) for displacing said image of said at least one slit (211) comprise :
   . a diaphragm (21) comprising said at least one slit (211),
   . a first lens (23), said at least one slit (211) being placed in the object focal plane of said first lens (23),
   . a second focusing lens (430) mobile in translation in said translation displacement direction.

22. Device according to claim 21, further comprising a rotating prism (420) located between said first and second lens (23,430) enabling a rotation of said translation displacement direction about an axis of said surface of said object (OE).

23. Device according to claim 22, wherein said means (4) for displacing said image of said at least one slit (211) enable displacement in translation of the latter in at least two orthogonal directions (OX,OY).

24. Device according to claim 23, for refractive eye surgery, wherein said surface of said objects (OE) the external face of the cornea (COR), and wherein to compensate for astigmatism of the eye by re-establishing the symmetry of revolution of said cornea (COR) said orthogonal directions (OX,OY) are respectively oriented along the principal directions of astigmatism.

25. Device according to claim 22, for refractive eye surgery, wherein said surface of said objects (OE) is the external face of the cornea (COR) and wherein, for treatment of myopia, said ablation function $A_u(u)$ satisfies the equation:

$$A_u(u) = 2A_u^0 \left( \frac{1}{2} - \frac{u^2}{R^2} \right)$$

in which equation:

$A_u^0$ is the maximum value of said ablation function,

R represents the radius of said cornea (COR).

26. Device according to claim 22, for refractive eye surgery, wherein said surface of said objects (OE) the external face of the cornea (COR) and wherein, for treatment of hypermetropia, said ablation function

$A_u(u)$ satisfies the equation:

$$A_u(u) = A_u^0 \frac{u^2}{v^2} \quad 0 \leqslant |u| \leqslant v \, (v < R)$$

in which equation:
$A_u^0$ is the maximum value of said ablation function,
R represents the radius of said cornea (COR),

27. Device according to claim 1, wherein said at least one slit (211) is formed on a diaphragm (21) forming a curved surface.

28. Device according to claim 27, wherein said diaphragm (21) comprises a semicylindrical surface of radius R with a longitudinal axis (O'''x), said at least one slit (211) having a profile function $E(\phi)$ in the direction O'''x, said plane with respect to a plane orthogonal to the longitudinal axis O'''x, said plane containing the directions O'''y and O'''z orthogonal to the direction of said longitudinal axis O'''x, said profile function $E(\phi)$ satisfying the equation:

$$E(\phi) = E\left(\tfrac{\pi}{2}\right) \sin^3\phi$$

in which equation:
- $\phi$ represents the angle of the radius vector of a point on the curved geometrical locus of symmetry of said at least one slit (211) and of said direction Oy, and
- $E(\pi/2)$ represents the maximal profile function of said at least one slit (211) for $\phi = \pi/2$.

29. Device according to claim 28 for refractive eye surgery wherein said surface of said objects (OE) is the external face of the cornea (COR) and wherein to compensate for astigmatism of the eye by re-establishing the symmetry of revolution of said cornea (COR) said diaphragm (21) has its concave side facing towards said cornea (COR) and said at least one slits (211) adapted to be illuminated by said laser beam (FL), said longitudinal axis O'''x and said transverse axis O'''y of said diaphragm (21) being oriented in the principal directions of astigmatism of said cornea (COR), said device further comprises drive means (400,401,402) for rotating said diaphragm (21) about said axis O'''y.

30. Device according to claim 29, further comprising drive means (400,401,402) for rotating said diaphragm (21) about said axis O'''y, consisting of a stepper motor enabling said diaphragm (21) to be rotated in rotation increments $\Delta\alpha$ satisfying the equation:

$$\Delta\alpha = \Delta 0 \left(1 - \frac{R^2}{Rx^2}\cos^2\alpha\right)^{-1}$$

in which equation:
$\alpha$      represents the inclination of said diaphragm (21) and of said axis O'''z relative to said reference direction OX, and
$\Delta 0$      represents the minimum angular rotation increment for $\alpha = \pi/2$,
R and $R_x$      are the radii of cornea (COR) respectively for $\alpha = 0$ and $\alpha = \pi/2$.

31. Device according to claim 30 further comprising means (8) for calculating the rotation translation displacement increment $\Delta\alpha$ which satisfies the equation:

$$\Delta\alpha = \Delta 0 (1 - \frac{R^2}{Rx^2} \cos^2\alpha)^{-1}$$

in which equation:

a          represents the inclination of said diaphragm (21) and of said axis O'''z relative to said reference direction OX, and

$\Delta 0$        represents the minimum angular rotation increment for $\alpha = \pi/2$,

R and $R_x$    are the radii of cornea (COR) respectively for $\alpha = 0$ and $\alpha = \pi/2$.

**Patentansprüche**

1. Einrichtung um die Form eines Gegenstandes (OE) durch Laser Ablation einer Oberfläche des Gegenstandes (OE) entsprechend einer Ablationsfunktion zu gestalten, wobei die Einrichtung beinhaltet:
   - Vorrichtungen (1) um einen gepulsten Laserstrahl zu erzeugen, der Pulse und eine Energiedichte hat,
   - Blendenvorrichtungen (21) mit einem wenigstens einmal vorhandenen Blendenausschnitt (211), der den Laserstrahl (FL) begrenzt und der wenigstens einmal vorhandene Blendenausschnitt (211) eine zu der Ablationsfunktion proportionale Profilfunktion hat,
   - Vorrichtungen (3), um den wenigstens einmal vorhandenen Blendenausschnitt (211) auf ein Gebiet auf der Oberfläche des Gegenstandes (OE) abzubilden,
   - Vorrichtungen (4) um das Bild des wenigstens einmal vorhandenen Blendenauschnitts (211) über das Gebiet, in Schritten eines gegebenen Inkrements, die elementaren diskreten Ablationen der Oberfläche des Gegenstandes (OE) entsprechen, zu verschieben,
   - Vorrichtungen (5), um das Inkrement, die Pulse und die Energiedichte aufeinander abzustimmen, so daß die gesamte Ablation, die sich aus der Summierung der elementaren diskreten Ablationen ergibt, der Ablationsfunktion genügt.

2. Einrichtung nach Anspruch 1, worin der wenigstens einmal vorhandene Blendenausschnitt (211) eine einstellbare Profilfunktion hat, um das Ausgleichen einer unregelmäßigen Verteilung der Energiedichte im Querschnitt des begrenzten Teils des Laserstrahls (FL) zu ermöglichen.

3. Einrichtung nach Anspruch 2, worin der wenigstens einmal vorhandene Blendenausschnitt (211) mindestens eine Kante aufweist, die aus Streifen (2110) aufgebaut ist, die translatorisch, in einer Richtung senkrecht zu der Längsachse des Blendenausschnitts (211), beweglich sind.

4. Einrichtung nach Anspruch 1, worin die Ablationsfunktion durch eine Funktion $A(h)$ bestimmt ist, die zu einer Achse (OZ) rotationssymmetrisch ist , die senkrecht zu der Oberfläche des Gegenstandes (OE) ist, wobei $h$ der Abstand zu dieser Achse (OZ) ist, und die Profilfunktion des wenigstens einmal vorhandenen Blendenausschnitts in einem Polarkoordinatensystem $(\rho,\theta)$ durch

$$\theta(\rho) = \frac{\Gamma}{\bar{a}(e)} A(\rho)$$

gegeben ist, wobei in der Gleichung:

$\Gamma$ ein Rotationswinkelinkrement ist,

$\bar{a}(e)$ die durchschnittliche Dicke darstellt, die durch die Bestrahlung durch jeden Laserpuls abgetragen wird.

5. Einrichtung nach Anspruch 4, worin eine anisotrope Ablationsfunktion $A(h,\beta)$ durch die Modulation des Rotationswinkelinkrements $\Gamma$ als eine Funktion des Azimutwinkels $\beta$ um die Achse (OZ) erreicht wird:

$$A(h, \beta) = \frac{\bar{a}(e)}{\Gamma(\beta)} \theta(h)$$

**6.** Einrichtung nach Anspruch 4, der weiterhin Vorrichtungen (8) aufweist, um das Rotationswinkelinkrement $\Gamma$ zu berechen das folgende Gleichung erfüllt:

$$\Gamma = \theta_{max} \frac{\bar{a}(e)}{A_0}$$

wobei in der Gleichung:

$\theta_{max}$ den maximalen Aperturwinkel der Profilfunktion des wenigstens einmal vorhandenen Blendenausschnitts (211) darstellt,

$A_0$ der maximale Wert der Ablationsfunktion ist.

**7.** Einrichtung nach Anspruch 4, die weiterhin Vorrichtungen (8) aufweist, um die Anzahl $NI_1$ der Laserpulse und die Anzahl der Rotationswinkelinkremente $\Gamma$ zu berechnen, wobei die Anzahl $NI_1$ der Pulse folgender Gleichung genügt:

$$NI_1 = ND_1 \frac{A_0}{\bar{a}(e)}$$

wobei in der Gleichung:

$ND_1$ die Anzahl der vollständig getrennten oder benachbarten Bilder des wenigstens einmal vorhandenen Blendenausschnitts (211) darstellt,

$A_0$ der maximale Wert der Ablationsfunktion ist.

**8.** Einrichtung nach Anspruch 7, die weiterhin Vorrichtungen (8) aufweist, um die minimale gesamte Bestrahlungszeit $T_{1min}$ zu berechnen, die der folgenden Gleichung genügt:

$$T_{1min} = NI_1 \frac{\tau(e)}{ND_1} = \tau(e) \frac{A_0}{\bar{a}(e)}$$

wobei in der Gleichung:

$\tau(e)$ das minimale Zeitintervall zwischen zwei aufeinanderfolgenden Bestrahlungen desselben Punkts des erwähnten Gebiets darstellen.

**9.** Einrichtung nach Anspruch 4, worin die Vorrichtungen (4), um das Bild des wenigstens einmal vorhandenen Blendenausschnittes (211) zu verschieben, beinhalten:

- Eine Blende (21) die den wenigstens einmal vorhandenen Blendenausschnitt (211) aufweist,
- Antriebsvorrichtungen, um die Blende (21) zu drehen, die aus einem gezahnten Ring (210), der auf dem Umfang der Blende (21) angebracht ist, und einem Schrittmotor (40) bestehen, dessen Antriebswelle mit wenigstens einem Zahnrad (41) verbunden ist, das mit dem gezahnten Ring (210) in Eingriff steht.

**10.** Einrichtung nach Anspruch 5 für operative Maßnahmen zum Ausgleich von Refraktionsfehlern, worin die Oberfläche des Gegenstandes (OE) die äußere Fläche der Hornhaut (COR) ist und worin die Einrichtung, um Astigmatismus des Auges durch die Wiederherstellung der Rotationssymmetrie der Hornhaut (COR) auszugleichen, Vorrichtungen (8, 5) aufweist, um das Rotationswinkelinkrement $\Gamma$ als eine Funktion des Azimutwinkels $\beta$ zu modulieren:

$$\Gamma(\beta) = \theta_{max} \frac{\bar{a}(e)}{A_0(\beta)}$$

wobei in der Gleichung:

$\theta_{max}$ den maximalen Aperturwinkel des Profils des wenigstens einmal vorhandenen Blendenausschnitts (211) darstellt,

$A_0(\beta)$ den maximalen Wert der Ablationsfunktion in der Richtung mit dem Azimutwinkel $\beta$ darstellt.

11. Einrichtung nach Anspruch 4 für operative Maßnahmen zum Ausgleich von Refraktionsfehlern, worin die Oberfläche des Gegenstandes (OZ) die äußere Fläche der Hornhaut (COR) ist und worin, um Astigmatismus des Auges durch die Wiederherstellung der Rotationssymmetrie der Hornhaut (COR) auszugleichen, die Vorrichtungen (3), um den wenigstens einmal vorhandenen Blendenausschnitt (211) abzubilden, ein anamorphes optisches System aufweisen, dessen Vergrößerung vom Azimutwinkel $\beta$ um die Achse (OZ) abhängt.

12. Einrichtung nach Anspruch 4 für operative Maßnahmen zum Ausgleich von Refraktionsfehlern, worin die Oberfläche des Gegenstandes (OE) die äußere Fläche der Hornhaut (COR) ist und worin, um Astigmatismus des Auges durch die Wiederherstellung der Rotationssymmetrie der Hornhaut (COR) auszugleichen, die Einrichtung wenigstens eine Hilfsblende mit einem Objektblendenausschnitt (211) in der Form eines Kreisbogens mit einem bestimmten Krümmungsradius aufweist.

13. Einrichtung nach Anspruch 4 für operative Maßnahmen zum Ausgleich von Refraktionsfehlern, worin die Oberfläche des Gegenstandes (OE) die äußere Fläche der Hornhaut (COR) ist und worin zur Behandlung von Myopie die Ablationsfunktion $A(h)$ folgender Gleichung genügt:

$$A(h) = A_0(1 - \frac{h^2}{R^2}) \quad 0 \le h \le R$$

wobei in der Gleichung:
   $A_0$ der maximale Wert der Ablationsfunktion ist.
   $R$ den Radius der Hornhaut (COR) darstellt.

14. Einrichtung nach Anspruch 4 für operative Maßnahmen zum Ausgleich von Refraktionsfehlern, worin die Oberfläche des Gegenstandes (OE) die äußere Fläche der Hornhaut (COR) ist und worin zur Behandlung von Hypermetropie die Ablationsfunktion $A(h)$ der folgenden Gleichung genügt:

$$A(h) = A_0 \frac{h^2}{\nu^2} \quad 0 \le h \le \nu \quad (\nu < R)$$

wobei in der Gleichung:
   $A_0$ der maximale Wert der Ablationsfunktion ist,
   $R$ den Radius der Hornhaut (COR) darstellt.

15. Einrichtung nach Anspruch 1, worin die Ablationsfunktion durch eine zylindrische Funktion $A_u(u)$ bestimmt ist, wobei $u$ eine Koordinate eines kartesischen Koordinaten Systems $(u,v)$ in einer zu der Oberfläche des Gegenstandes (OE) tangentialen Fläche ist und die Profilfunktion des wenigstens einmal vorhandenen Blendenausschnitts (211) in einem kartesischen Koordinaten System $(E,z)$ gegeben ist durch:

$$E(z) = \frac{\Delta_v}{\bar{a}_u(e)} A_u(z)$$

wobei in der Gleichung:

$\Delta_v$ ein Inkrement der translatorischen Verschiebung in der *v* Richtung ist,

$\bar{a}_u(e)$ die durchschnittliche Dicke darstellt, die durch Bestrahlung durch jeden Laserpuls in der *v* Richtung abgetragen wird.

16. Einrichtung nach Anspruch 15, worin der wenigstens einmal vorhandene Blendenausschnitt (211) eine einstellbare Profilfunktion hat, um das Ausgleichen einer unregelmäßigen Verteilung der Energiedichte im Querschnitt des begrenzten Teils des Laserstrahls (FL) zu ermöglichen.

17. Einrichtung nach Anspruch 16, worin der wenigstens einmal vorhandene Blendenausschnitt (211) mindestens eine Kante aufweist, die aus Streifen (2110) aufgebaut ist, die translatorisch beweglich sind.

18. Einrichtung nach Anspruch 15, die weiterhin Vorrichtungen (8) aufweist, um das Inkrement der translatorischen Verschiebung $\Delta_v$ zu berechnen, das der folgenden Gleichung genügt: wobei in der Gleichung:

$$\Delta_v = E_{max} \frac{\bar{a}_u(e)}{A_u^0}$$

$E_{max}$ die maximale Weite der Profilfunktion des wenigstens einmal vorhandenen Blendenausschnitts (211) darstellt,

$$A_u^0$$

der maximale Wert der Ablationsfunktion ist.

19. Einrichtung nach Anspruch 15, die weiterhin Vorrichtungen (8) aufweist, um die Anzahl

$$NI_2^v$$

der Laserpulse und die Anzahl der Inkremente $\Delta_v$ der translatorischen Verschiebung in der *v* Richtung zu berechnen, wobei die Anzahl

$$NI_2^v$$

der Pulse der folgenden Gleichung genügt:

$$NI_2^v = ND_2^v \frac{A_u^0}{\bar{a}_u(e)}$$

wobei in der Gleichung:

$$ND_2^v$$

die Anzahl der vollständig getrennten oder benachbarten Bilder des wenigstens einmal vorhandenen Blendenausschnitts (211) in der *v* Richtung ist,

$$A_u^0$$

der maximale Wert der Ablationsfunktion ist.

20. Einrichtung nach Anspruch 19, die weiterhin Vorrichtungen (8) aufweist, um die minimale gesamte Bestrahlungszeit

$$T_{2min}^{v}$$

in der *v* Richtung zu berechnen, die folgender Gleichung genügt:

$$T_{2min}^{v} = NI_2^{v}\frac{\tau^{v}(e)}{ND_2^{v}} = \tau^{v}(e)\frac{A_u^0}{\bar{a}_u(e)}$$

wobei in der Gleichung:
$\tau^{v}(e)$ das minimale Zeitintervall zwischen zwei aufeinanderfolgenden Bestrahlungen desselben Punktes auf dem vorerwähnten Gebiet in der *v* Richtung darstellt.

21. Einrichtung nach Anspruch 15, worin die Vorrichtungen (4) zur Verschiebung des Bildes des wenigstens einmal vorhandenen Blendenausschnitts (211) beinhalten:
   - Eine Blende (21), die den wenigstens einmal vorhandenen Blendenausschnitt (211) aufweist,
   - eine erste Linse (23), wobei der wenigstens einmal vorhandene Blendenausschnitt (211) in der Brennebene der ersten Linse (23) angebracht ist,
   - eine zweite Fokussierungslinse (430), die in der translatorischen Verschiebungsrichtung translatorisch beweglich ist.

22. Einrichtung nach Anspruch 21, die weiterhin ein rotierendes Prisma (420) aufweist, das zwischen der ersten und zweiten Linse (23, 430) angebracht ist und eine Drehung der translatorischen Verschiebungsrichtung um eine Achse der Oberfläche des Gegenstandes (OE) ermöglicht.

23. Einrichtung nach Anspruch 22, worin die Vorrichtungen (4), zur Verschiebung des Bilds des wenigstens einmal vorhandenen Blendenausschnitts (211), die translatorische Verschiebung des letzteren in wenigstens zwei orthogonale Richtungen (OX, OY) ermöglichen.

24. Einrichtung nach Anspruch 23 für operative Maßnahmen zum Ausgleich von Refraktionsfehlern, worin die Oberfläche des Gegenstandes (OE) die äußere Fläche der Hornhaut (COR) ist und worin, um Astigmatismus des Auges durch Wiederherstellung der Rotationssymmetrie der Hornhaut (COR) auszugleichen, die orthogonalen Richtungen (OX, OY) jeweils entlang der Hauptrichtungen des Astigmatismus orientiert sind.

25. Einrichtung nach Anspruch 22 für operative Maßnahmen zum Ausgleich von Refraktionsfehlern, worin die Oberfläche des Gegenstandes (OE) die äußere Fläche der Hornhaut (COR) ist und worin zur Behandlung von Myopie die Ablationsfunktion $A_u(u)$ folgender Gleichung genügt:

$$A_u(u) = 2A_u^0\left(\frac{1}{2} - \frac{u^2}{R^2}\right)$$

wobei in der Gleichung:

$$A_u^0$$

der maximale Wert der Ablationsfunktion ist,
   $R$    den Radius der Hornhaut (COR) darstellt.

33

**26.** Einrichtung nach Anspruch 22 für operative Maßnahmen zum Ausgleich von Refraktionsfehlern, worin die Oberfläche des Gegenstandes (OE) die äußere Fläche der Hornhaut (COR) ist und worin zur Behandlung von Hypermetropie die Ablationsfunktion $A_u(u)$ folgender Gleichung genügt:

$$A_u(u) = A_u^0 \frac{u^2}{\nu^2} \quad 0 \leq |u| \leq \nu \quad (\nu < R)$$

wobei in der Gleichung:

$$A_u^0$$

der maximale Wert der Ablationsfunktion ist,
$R$     den Radius der Hornhaut darstellt.

**27.** Einrichtung nach Anspruch 1, worin der wenigstens einmal vorhandene Blendenausschnitt (211) auf einer Blende (21) ausgebildet ist, die eine gekrümmte Oberfläche bildet.

**28.** Einrichtung nach Anspruch 27, worin die Blende (21) eine halbzylindrische Oberfläche mit dem Radius $R$ mit einer Längsachse (O'''x) aufweist, der wenigstens einmal vorhandene Blendenausschnitt (211) eine Profilfunktion $E(\phi)$ in der Richtung O'''x hat, die Ebene, die die Richtungen O'''y und O'''z enthält orthogonal zu der Richtung der Längsachse O'''x ist und die Profilfunktion $E(\phi)$ die folgende Gleichung erfüllt:

$$E(\phi) \;=\; E(\tfrac{\pi}{2})\sin^3\phi$$

wobei in der Gleichung:
$\phi$ den Winkel des Radiusvektors eines Punktes auf dem gekrümmten Symmetrieort des wenigstens einmal vorhandenen Blendenausschnitts (211) und der Richtung OY darstellt, und

$$E(\tfrac{\pi}{2})$$

die maximale Profilfunktion des wenigstens einmal vorhandenen Blendenausschnitts (211) für $\phi = \tfrac{\pi}{2}$ darstellt.

**29.** Einrichtung nach Anspruch 28 für operative Maßnahmen zum Ausgleich von Refraktionsfehlern, worin die Oberfläche des Gegenstandes (OE) die äußere Fläche der Hornhaut (COR) ist und worin, um Astigmatismus des Auges durch die Wiederherstellung der Rotationssymmetrie der Hornhaut (COR) auszugleichen, die Blende (21) ihre konkave Seite der Hornhaut (COR) zugewandt hat, und der wenigstens einmal vorhandene Blendenausschnitt (211) vorgesehen ist, von dem Laserstrahl (FL) beleuchtet zu werden, die Längsachse O'''x und die Querachse O'''y der Blende (21) in den Hauptrichtungen des Astigmatismus der Hornhaut (COR) orientiert sind und die Vorrichtung weiterhin Antriebsvorrichtungen (400, 401, 402) aufweist, um die Blende (21) um die Achse O'''y zu drehen.

**30.** Einrichtung nach Anspruch 29, die weiterhin Antriebsvorrichtungen (400, 401, 402) um die Blende (21) um die Achse O'''y zu drehen, aufweist, die aus einem Schrittmotor bestehen, der es ermöglicht die Blende (21) zu drehen, in Inkrementen der Drehung $\Delta\alpha$, die die folgende Gleichung erfüllen:

$$\Delta\alpha = \Delta O\left(1 - \frac{R^2}{R_x^2}cos^2\alpha\right)^{-1}$$

wobei in der Gleichung:

$\alpha$ die Inklination der Blende (21) und der Achse O'''z, relativ zu der Referenzrichtung OX darstellt, und
$\Delta O$ das minimale Winkelinkrement der Drehung für $\alpha = \frac{\pi}{2}$ darstellt,
$R$ und $R_x$ die Radien der Hornhaut (COR) jeweils für $\alpha = 0$ und $\alpha = \frac{\pi}{2}$ sind.

31. Einrichtung nach Anspruch 30, die weiterhin Vorrichtungen (8) aufweist, um das Inkrement der Drehung $\Delta\alpha$ zu berechnen, das die folgende Gleichung erfüllt:

$$\Delta\alpha = \Delta O(1 - \frac{R^2}{R_x^2}cos^2\alpha)^{-1}$$

wobei in der Gleichung:
$\alpha$ die Inklination der Blende (21) und der Achse O'''z, relativ zu der Referenzachse OX darstellt, und
$\Delta O$ das minimale Winkelinkrement der Drehung für $\alpha = \frac{\pi}{2}$ darstellt,
$R$ und $R_x$ die Radien der Hornhaut (COR) jeweils für $\alpha = 0$ und $\alpha = \frac{\pi}{2}$ sind.

## Revendications

1. Dispositif de mise en forme d'un objet (OE) par ablation par laser d'une surface dudit objet (OE) selon une fonction d'ablation, ledit dispositif comprenant:

un moyen (1) de génération d'un faisceau laser pulsé (FL) à impulsions et à haute densité d'énergie,

un moyen de fente (21) comprenant au moins une fente (21) interceptant ledit faisceau laser (FL), ladite fente au moins unique (211) exerçant une fonction de profil proportionnelle à ladite fonction d'ablation,

un moyen (3) de formation d'une image de ladite fente au moins unique (211) sur une zone de ladite surface dudit objet (OE)

un moyen (4) de déplacement de ladite image de ladite fente au moins unique (211) sur ladite zone, selon des pas d'un incrément donné, correspondant à des ablations discrètes élémentaires de ladite surface dudit objet (OE),

un moyen (5) de synchronisation dudit incrément, desdites impulsion et de ladite densité d'énergie, d'une manière telle que l'ablation totale résultant de la sommation desdites ablations discrètes élémentaires corresponde à ladite fonction d'ablation.

2. Dispositif selon la revendication 1 dans lequel ladite fente au moins unique (211) présente une fonction réglable de profil afin de permettre une compensation d'une répartition irrégulière de ladite densité d'énergie dans la section transversale de la partie interceptée dudit faisceau laser (FL).

3. Dispositif selon la revendication 2 dans lequel ladite fente au moins unique (211) comprend au moins un bord constitué de bandes (2110) mobiles en translation dans une direction perpendiculaire à l'axe longitudinale de ladite fente (211).

4. Dispositif selon la revendication 1, dans lequel, ladite fonction d'ablation étant définie par une fonction A(h) à symétrie de révolution autour d'un axe perpendiculaire à ladite surface dudit objet (OE), h étant la distance audit axe (OZ), ladite fonction de profil de ladite fente au moins unique (211) dans un système de coordonnées polaires ($\rho$, $\Theta$) est donnée par

$$\Theta(\rho) = \frac{\Gamma}{a(e)} \, A(\rho)$$

équation dans laquelle
$\Gamma$ est un incrément de rotation angulaire
a(e) représente l'épaisseur moyenne enlevée par irradiation par chaque impulsion laser.

5. Dispositif selon la revendication 4 dans lequel une fonction anisotropique d'ablation A (h, $\beta$) est obtenue en modulant ledit incrément de rotation angulaire $\Gamma$ en fonction d'un angle azimutal $\beta$ autour dudit axe

(Oz):

$$A(h, \beta) = \frac{\overline{a(e)}}{\Gamma(\beta)} \Theta(h)$$

6. Dispositif selon la revendication 4, comprenant en outre un moyen de calcul (8) de l'incrément $\Gamma$ de rotation angulaire qui satisfait l'équation:

$$\Gamma = \Theta_{max} \frac{\overline{a(e)}}{A_0}$$

équation dans laquelle

$\Theta_{max}$ représente l'angle maximal d'ouverture de ladite fonction de profil de ladite fente au moins unique (211),

$A_0$ est la valeur maximale de ladite fonction d'ablation.

7. Dispositif selon la revendication 4, comprenant en outre un moyen de calcul (8) du nombre $NI_l$ d'impulsion laser et le nombre d'incrément $\Gamma$ de rotation, le nombre $NI_l$ d'impulsions satisfaisant à l'équation:

$$NI_1 = N_{DI} \frac{A_0}{\overline{a(e)}}$$

équation dans laquelle

$ND_l$ représente le nombre d'images totalement séparées ou adjacentes de ladite fente au moins unique (211);

$A_0$ est la valeur maximale de ladite fonction d'ablation.

8. Dispositif selon la revendication 7, comprenant en outre un moyen de calcul (8) du temps minimal total d'irradiation $T_{lmin}$ qui satisfait à l'équation

$$T_{Imin} = NI_1 \frac{\tau(e)}{N_{DI}} = \tau(e) \frac{A_0}{\overline{a(e)}}$$

équation dans laquelle

$\tau(e)$ représente l'intervalle minimal de temps entre deux irradiations successives au même point de la dite zone.

9. Dispositif selon la revendication 4, dans lequel lesdits moyens (4) de déplacement de ladite image de ladite fente au moins unique (211) comprennent:

un diaphragme (21) comportant ladite fente au moins unique (211);

un moyen d'entraînement servant à faire tourner le diaphragme (21), comprenant une couronne dentée (210) disposée à la périphérie dudit diaphragme (21) et un moteur pas-à-pas (40) dont l'arbre d'entraînement est équipé d'au moins une roue dentée (41) qui s'engrène avec ladite couronne dentée (210).

10. Dispositif selon la revendication 5, servant à une chirurgie réfractive de l'oeil, dans lequel ladite surface dudit objet (OE) est la face externe de la cornée (COR) et dans lequel ledit dispositif comprend, afin de compenser un astigmatisme de l'oeil en rétablissant la symétrie de révolution de ladite cornée (COR), un moyen de modulation dudit incrément $\Gamma$ de rotation angulaire en fonction dudit angle azimutal $\beta$:

EP 0 296 982 B1

$$\Gamma\ (\beta)\ =\ \Theta_{max}\ \frac{\bar{a}(e)}{A_0(\beta)}$$

équation dans laquelle

$\Theta_{max}$ représente l'angle maximal d'ouverture dudit profil de ladite fente au moins unique (211)

$A_0(\beta)$ représente la valeur maximale de ladite fonction d'ablation dans la direction de l'angle azimutal $\beta$.

11. Dispositif selon la revendication 4, servant à une chirurgie réfractive de l'oeil, dans lequel ladite surface dudit objet (OE) est la face externe de la cornée (COR) et dans lequel ledit moyen de formation d'une image de ladite fente au moins unique (211) comprend, afin de compenser un astigmatisme de l'oeil en rétablissant la symétrie de révolution de ladite cornée (COR), un système optique anamorphique (9) dont le grossissement dépend de l'angle azimutal ($\beta$) autour dudit axe Oz.

12. Dispositif selon la revendication 4, servant à une chirurgie réfractive de l'oeil, dans lequel ladite surface dudit objet (OE) est la face externe de la cornée (COR) et dans lequel ledit dispositif comprend, afin de compenser un astigmatisme de l'oeil en rétablissant la symétrie de révolution de ladite cornée (COR), au moins un diaphragme auxiliaire à fente objet (211) en forme d'arc circulaire à rayon de courbure spécifique.

13. Dispositif selon la revendication 4, servant à une chirurgie réfractive de l'oeil, dans lequel ladite surface dudit objet (OE) est la face externe de la cornée (COR) et dans lequel ladite fonction d'ablation A(h) satisfait, pour le traitement de la myopie, à l'équation:

$$A(h)\ =\ A_0\ (1\ -\ \frac{h^2}{R^2})\qquad\qquad 0\ \leq\ h\ \leq\ R$$

équation dans laquelle

$A_0$ est la valeur maximale de ladite fonction d'ablation,

R représente le rayon de ladite cornée (COR)

14. Dispositif selon la revendication 4, servant à une chirurgie réfractive de l'oeil, dans lequel ladite surface dudit objet (OE) est la face externe de la cornée (COR) et dans lequel ladite fonction d'ablation A(h) satisfait, pour le traitement de hypermétropie, à l'équation :

$$A(h)\ =\ A_0\ \frac{h^2}{\nu^2}\qquad\qquad 0\ \leq\ h\ \leq\ \nu\quad (\ \nu\ <\ R)$$

équation dans laquelle

$A_0$ est la valeur maximale de ladite fonction d'ablation,

R représente le rayon de ladite cornée (COR)

15. Dispositif selon la revendication 1, dans lequel ladite fonction d'ablation est définie par une fonction cylindrique $A_u(u)$, u étant une coordonnée d'un système de coordonnées cartésiennes, (u, v) dans un plan tangentiel à ladite surface dudit objet (OE), ladite fonction de profil de ladite fente au moins unique (211) dans un système de coordonnées cartésiennes (E, z) est donné par:

$$E(z)\ =\ \frac{\Delta v}{\bar{a}_u(e)}\ A_u(z)$$

équation dans laquelle

$\Delta v$ est un incrément de déplacement en translation dans la direction v.

$\bar{a}_u(e)$ représente l'épaisseur moyenne enlevée par un rayonnement de chaque impulsion laser dans la direction u.

37

EP 0 296 982 B1

16. Dispositif selon la revendication 15, dans lequel ladite fente au moins unique (211) présente une fonction réglable de profil afin de permettre une compensation d'une répartition irrégulière de ladite densité d'énergie dans la section transversale de la partie interceptée dudit faisceau laser (FL).

17. Dispositif selon la revendication 16, dans lequel ladite fente au moins unique (211) comprend au moins un bord constitué de bandes (2110) mobiles en translation.

18. Dispositif selon la revendication 15, comprenant en outre un moyen de calcul (8) de l'incrément $\Delta v$ de déplacement de translation qui satisfait à l'équation

$$\triangle v = E_{max} \frac{\bar{a}_u(e)}{A_u^0}$$

équation dans laquelle
$E_{max}$ représente la largeur maximale de ladite fonction de profil de ladite fente au moins unique (211)
$A_u^0$ est la valeur maximale de ladite fonction d'ablation.

19. Dispositif selon la revendication 15, comprenant en outre un moyen de calcul (8) du nombre $NI^v_2$ l'impulsion laser et le nombre de déplacement en translation v dans la direction v, le nombre $NI^v_2$ d'impulsions satisfaisant à l'équation:

$$NI^V_2 = ND^V_2 \frac{A_u^0}{\bar{a}_u(e)}$$

équation dans laquelle
$ND^v_2$ représente le nombre d'images totalement séparées ou adjacentes de ladite fente au moins unique (211) dans la direction v,
$A_u^0$ est la valeur maximale de ladite fonction d'ablation.

20. Dispositif selon la revendication 19, comprenant en outre, un moyen de calcul (8) du temps minimal total d'irradiation $T^v_{2min}$ dans la direction v qui satisfait l'équation:

$$T^V_{2min} = NI^V_2 \frac{\tau^V(e)}{ND^V_2} = \tau^V(e) \frac{A_u^0}{\bar{a}_u(e)}$$

équation dans laquelle
$\tau^v(e)$ représente l'intervalle de temps minimal entre deux irradiations successives d'un même point de ladite zone dans la direction v.

21. Dispositif selon la revendication 15, dans lequel ledit moyen (4) de déplacement de ladite image de ladite fente au moins unique (211) comprend:
un diaphragme (21) comportant ladite fente au moins unique (211),
une première lentille (23), ladite fente au moins unique (211) étant placée dans le plan focal objet de ladite première lentille (23)
une deuxième lentille de focalisation (430) mobile en translation dans ladite direction de déplacement en translation.

22. Dispositif selon la revendication 21, comprenant en outre un prisme tournant (426) positionné entre ladite première et ladite deuxième lentilles (23, 430), permettant une rotation de ladite direction de déplacement en translation autour d'un axe de ladite surface dudit objet (OE).

23. Dispositif selon la revendication 22, dans lequel ledit moyen (4) de déplacement de ladite image de ladite fente au moins unique (211) permet un déplacement en translation de cette dernière dans au moins deux directions orthogonales (OX, OY).

38

**24.** Dispositif selon la revendication 23, servant à une chirurgie réfractive de l'oeil, dans lequel ladite surface dudit objet (OE) est la face externe de la cornée (COR) et dans lequel lesdites direction orthogonales (Ox, Oy) sont respectivement orientées selon les directions principales d'astigmatisme afin de compenser un astigmatisme de l'oeil en rétablissant la symétrie de révolution de ladite cornée (COR).

**25.** Dispositif selon la revendication 22, servant à une chirurgie réfractive de l'oeil, dans lequel ladite surface dudit objet (OE) est la face externe de la cornée (COR) et dans lequel ladite fonction d'ablation $A_u(u)$ satisfait, pour le traitement de la myopie, à l'équation:

$$A_u(u) = 2A_u^0 \left(\frac{1}{2} - \frac{u^2}{R^2}\right)$$

équation dans laquelle $A_u^0$ est la valeur maximale de ladite fonction d'ablation,
R représente le rayon de ladite cornée (COR)

**26.** Dispositif selon la revendication 22, servant à une chirurgie réfractive de l'oeil, dans lequel ladite surface dudit objet (OE) est la face externe de la cornée (COR) et dans lequel ladite fonction d'ablation A(h) satisfait, pour le traitement de hypermétropie, à l'équation :

$$A_u(u) = A_u^0 \frac{u^2}{v^2} \qquad 0 \leq |u| \leq v \quad (v < R)$$

équation dans laquelle
$A_u^0$ est la valeur maximale de ladite fonction d'ablation,
R représente le rayon de ladite cornée (COR)

**27.** Dispositif selon la revendication 1, dans lequel ladite fente au moins unique (211) est formée sur un diaphragme (21) formant une surface incurvée.

**28.** Dispositif selon la revendication 27, dans lequel ledit diaphragme (21) comprend une surface semi-cylindrique de rayon R d'axe longitudinal (O'''z), ladite fente au moins unique possédant une fonction de profil $E(\phi)$ dans la direction O'''x, ledit plan par rapport à un plan orthogonal à la direction longitudinal (O'''x), ledit plan contenant les direction (O'''y) et (O'''z) orthogonales à la direction dudit axe longitudinal (O'''x), ladite fonction de profit $E(\phi)$ satisfaisant à l'équation:

$$E(\phi) = E\left(\tfrac{\pi}{2}\right) \sin^3 \phi$$

équation dans laquelle
$\phi$ représente l'angle du vecteur de rayon d'un point du lieu géométrique incurvé de symétrie de ladite fente au moins unique (211) et de ladite direction Oy, et,
$E(\pi/2)$ représente la fonction maximale de profil de ladite fente au moins unique pour $\phi = \pi/2$.

**29.** Dispositif selon la revendication 28, destiné à une chirurgie réfractive de l'oeil, dans lequel ladite dudit objet (OE) est la face externe de la cornée (COR) et dans lequel, afin de compenser un astigmatisme de l'oeil en rétablissant la symétrie de révolution de ladite cornée (COR), le côté concave du diaphragme (211) est tourné vers ladite cornée (COR) et ladite fente au moins unique (211) est apte à être illuminée par ledit faisceau laser (FL), ledit axe longitudinal O'''x et ledit axe transversal O'''y dudit diaphragme (21) étant orientés dans les directions principales d'astigmatisme de ladite cornée (COR), ledit dispositif comprend en outre un moyen d'entraînement (400, 401, 402) pour faire tourner ledit diaphragme (21) autour dudit axe O'''y.

**30.** Dispositif selon la revendication 29, comprenant en outre un moyen d'entraînement (400, 401, 402) pour faire tourner ledit diaphragme (21) autour dudit axe O'''y, consistant en un moteur pas-à-pas, permettant audit diaphragme (21) d'être tourné par des incréments de rotation $\Delta\alpha$ satisfaisant à

l'équation

$$\triangle\alpha = \triangle O(1 - \frac{R^2}{Rx^2} \cos^2 \alpha)^{-1}$$

équation dans laquelle
$\alpha$ représente l'inclinaison dudit diaphragme (21) et dudit axe O'''z par rapport à ladite direction de référence OX, et
$\Delta O$ représente l'incrément minimal de rotation pour $\alpha = \pi/2$,
R et Rx sont les rayons de la cornée (COR) respectivement pour $\alpha = 0$ et $\alpha = \pi/2$.

31. Dispositif selon la revendication 30 comprenant en outre un moyen de calcul (8) de l'incrément $\Delta\alpha$ de déplacement de translation de rotation qui satisfait à l'équation

$$\triangle\alpha = \triangle O(1 - \frac{R^2}{Rx^2} \cos^2 \alpha)^{-1}$$

équation dans laquelle
$\alpha$ représente l'inclinaison dudit diaphragme (21) et dudit axe O'''z par rapport à ladite direction de référence OX, et
$\Delta O$ représente l'incrément minimal de rotation pour $\alpha = \pi/2$,
R et Rx sont les rayons de la cornée (COR) respectivement pour $\alpha = 0$ et $\alpha = \pi/2$.

40

Elementary ablation depth per pulse

193 nm

FIG.1

FIG.2a

FIG.2b

FIG.3a

FIG_3b

FIG_3c

FIG_3d

FIG_3e

FIG_3f

FIG.4a

FIG_4b

FIG_4c

$211_1$   $211_2$   $211_3$

21

FIG.4d

21

DL

211

0″

d

z

2110   2111

FIG.4e

Y

+Ry   Ce

G   E

F   H

A

-Rx   +Rx

0   X

I

-Ry

FIG.5a

Centre of the cornea     Radius h

## FIG.5b

Centre of the cornea     Radius h

## FIG.5c

FIG.6a

FIG.6b

FIG. 6c

FIG. 6d

FIG. 6e

51

FIG.7